# EUROPEAN PATENT APPLICATION

(11) **EP 1 637 540 A1**
(43) Date of publication of application: **22.03.2006**
(21) Application number: 04077629.6
(22) Date of filing: 17.09.2004
(51) Int. Cl.: C07K 14/47, A61K 38/17, G01N 33/50, C12N 15/01

(54) **Hyperactive Stat molecules and their use in assays employing gene activation**

(71) Applicant: Forschungsverbund Berlin e.V., 12489 Berlin (DE)
(72) Inventor: Meyer, Thomas, Prof., 34225 Baunatal (DE); Vinkemeier, Uwe, Dr., 13189 Berlin (DE)
(74) Representative: Hertin, Paul W.

(57) **Abstract**

The invention relates to Stat molecule occurring in the nucleus of a cutaneous T cell lymphoma cell line which are characterized in that at least one conserved hydrophobic amino acid on the surface of the N-domain of the Stat wild type is mutated. The mutated phenotype bears a dephosphorylation and tetramerization defect, thereby prolonging Stat accumulation in the nucleus and affecting transcription in a promoter-specific manner. The invention also relates to the use of the mutated Stat molecules in the activation of gene expression, in the screening of inhibitors or activators of transcription factors, the identification of binding proteins, the production of a drug for diagnosis and/or treatment of diseases associated with the activation of gene expression as well as in the targeting of active substances.

## Description

The invention relates to Stat molecules which are mutated and their use in the activation of gene expression, in the screening of inhibitors or activators of transcription factors, the identification of binding proteins, the production of a drug for diagnosis and/or treatment of diseases associated with the activation of gene expression as well as in the targeting of active substances.

In eukaryotic organisms, DNA replication and RNA biogenesis take place in the cell nucleus, whereas protein synthesis essentially proceeds in the cytoplasm. The close integration of these activities depends on a selective transport of the proteins and ribonucleoprotein particles between nucleus and cytoplasm requiring a coupled import and export of compounds at many molecular processes. This translocation of proteins, DNA and/or RNA proceeds via nuclear pores which are located in the membrane enclosing the cell nucleus. Since the translocation is mediated, *inter* alia, via distinct signals, the process is termed signal transduction.
Whereas information and studies relating to the transport of proteins into the nucleus are available, there is only little information regarding the accumulation and the export of proteins and/or peptides or other biological structures out of the cell nucleus.

For example, the Stat (signal transducers and activators of transcription) proteins constitute an ancient and evolutionary conserved family of transcription factors involved in the regulated expression of numerous target genes in response to cytokines and growth factors (Darnell, J. E., Jr., Science 277, 1630-1635 (1997)). Polypeptide growth factors and cytokines elicit their effects by activating specific cell-surface receptors, thereby initiating signaling cascades. Canonical signaling through the Janus tyrosine kinase (Jak) / Stat pathway begins at the cell membrane within the cytoplasm. The binding of the mentioned ligands to their cognate receptors initiates a number of tyrosine phosphorylations catalyzed by Jak. The bound Stat monomers detach from their receptor docking sites after phosphorylation of a single tyrosine residue at their C-terminus finally forming reciprocal homo- or heterodimers. This sequence of events is commonly referred to as "Stat activation" triggering the accumulation of Stat dimers in the nucleus within a certain period. Eventually, they induce transcription by binding to palindromic DNA recognition sites named γ-activated sequence (GAS).

The mechanisms that control the predominantly cytoplasmic localization in unstimulated cells have not been resolved.
The translocation of protein substrates across the nuclear envelope commonly involves transport factors that share homology with the transport factor p97 (also called importin-β). These proteins mediate passage of protein cargoes through the nuclear pore complex (NPC). Based on the direction of cargo transport they have been classified as importins or exportins. The transport process requires metabolic energy and it is propelled by a concentration gradient across the nuclear membrane of the GTP-bound form of the G-protein Ran, which is found predominantly in the nucleus. The transport substrates are distinguished in their amino acid sequence by the presence of cis-acting nuclear localization signals (NLS) and/or nuclear export signals (NES). Recently, such transport signals were also identified in the Stats. These proteins contain canonical leucine-rich export signals which are required for binding to the exportin CRM1. Since pharmacological or mutational inactivation of the NES/CRM1 pathway does not preclude nuclear export, further transport mechanisms for the removal of Stat1 from the nucleus remain to be characterized. In addition, the DNA binding domain of the Stat1 molecule harbors a dimer-specific NLS (dsNLS) that constitutes the binding surface for the adaptor protein NPI-1 of the importin-α family, which tethers the Stat1 protein to the nuclear import factor p97 in a Ran-dependent manner. Destruction of the dsNLS precludes nuclear translocation of phosphorylated Stat1 and thus results in the loss of cytokine-inducible transcriptional responses.

It was shown that Stat proteins can leave the nucleus only after tyrosine dephosphorylation, and DNA binding was identified as the critical regulator of the conversion (Meyer *et al*., Genes Dev. 17, 1992-2005 (2003)). However, at present the physiological interfaces that modulate cooperative DNA binding and tyrosine dephosphorylation of Stat proteins are poorly defined.

Packing in the Stat4 crystal suggested two interfaces (I and II) that are potentially relevant for dimer formation. Previous biochemical investigations concerning mutations of an invariant tryptophane residue in position 37 of the N-domain led to the loss of cooperative Stat binding to tandem sites on DNA. Consequently, the crystal structure was interpreted in terms of the putative dimer interface I including the amino acid tryptophane.

Due to further experimental studies, the alternate surface ― termed interface II - was proposed to mediate Stat dimerization as requirement for the formation of higher order Stat complexes on DNA (Chen *et al.,* Protein Science 12, 361-365 (2003)). These studies analyzed point mutations which were introduced in the Stat1 N-domain at several sites of each interface. The dimerization properties of these mutated proteins were determined by analytical ultracentrifugation and gel filtration. Among others, the mutant Stat1 F77A characterized by a phenylalanine-to-alanine substitution on interface II was investigated and shown to be a monomer. Beside the fact that interface II is more extensive than interface I, it also involves interactions between hydrophobic residues probably affected by the replacement of phenylalanine. Referring to the mentioned tryptohane mutant, the new results reflect a non-specific domain destabilization rather than a specific defect in dimerization.

Unfortunately, no more biochemical and structural information are available about these interface interactions and their role within the N-domain for dimerization.
In particular, Stat proteins regulate many of the genes controlling cellular functions like cellular growth, differentiation and survival. Given the importance of Stats in the control of normal physiological processes, it is self-evident that inappropriate activation of these proteins is found in various disease patterns. Usually, cytokine-induced transcription is a transitory process which can take from a few minutes to hours. Cells with a deviantly high and prolonged Jak or Stat activation are susceptible to transformations and associated with abnormal developments (Frank, D. A., Mol. Med. 5, 432-456 (1999)). Interferences in the signaling pathways are the crucial factor for the appearance of pathogenic phenotypes such as human malignancies, leukemia, disorders in cell growth, defects of the immune system or in the metabolic performance of liver, kidneys and mammary gland, septic shock, arthritis, numerous cardiovascular diseases and other things.

The tack of more detailed information on the pathogenic mechanisms of signal transduction is particularly disadvantageous since current therapeutic strategies, for example to treat cancer, are both less selective and fairly toxic.

Therefore, the technical problem forming the basis of the present invention is to provide agents which modify the transcription activity of target genes and to provide agents and methods for the screening and targeting of active substances which modify the transcription activity, so that they are suitable in the diagnosis and/or treatment of diseases associated with the abnormal Stat induction of target genes.

The present invention solves this problem by providing a Stat molecule occurring in the nucleus of a cutaneous T cell lymphoma cell line which is characterized in that at least one conserved hydrophobic amino acid on the surface of the N-domain of the Stat wild type is mutated.

The formation of Stat tetramers and higher order oligomers on DNA results from cooperative DNA binding of Stat dimers that appears to be conserved throughout the Stat family. Generally, Stat proteins consist of a large core domain including the Src homology domain 2 (SH2) responsible for dimer formation and the DNA binding module which denotes an amino acid sequence capable of binding to a specific DNA sequence. The core is connected through a proteolytically cleavable peptide to the smaller N-domain which engages in a multitude of protein-protein interactions. This N-domain mediates cooperative DNA binding of Stat dimers. Several Stat molecules (Stat1, Stat3, Stat4, Stat5, Stat6) are able to form higher order complexes (dimer-dimer or higher) on promoters containing two or more neighboring Stat binding sites. Likely, Stat tetramers are also mandatory for full transcription activity at many promoters that contain only a single (not optimal) canonical GAS site.

The term "promoter" refers to a DNA sequence, mostly situated upstream (5') of the coding sequence of a structural gene which controls the expression of the coding region by providing a recognition sequence for an RNA polymerase and/or other factors required to start transcription at the appropriate position. Promoter sequences are necessary, but not sufficient in any case, to control expression of the gene.

The mutation of a conserved residue located in the alternate surface does not compromise protein stability, but causes a severe decrease in tetramer formation of several Stat molecules without overtly affecting the DNA binding of the dimer.

Importingly, the novel penotype of the mutants makes insights possible into the role that DNA binding plays in nuclear accumulation of Stat proteins. It was previously hypothesized that the access to a nuclear export signal (NES) within the coiled-coil domain is regulated by DNA binding. According to this model, the Stat association to DNA would mask the NES and thus preclude nuclear export. Consequently, the loss of DNA binding would result in the loss of nuclear accumulation. The novel phenotype of the mutants actually reveals that it is dimerization only (and not the subsequent DNA binding) that masks the region required for the nuclear export of Stat proteins. Thus, DNA binding is not necessary, but sufficient for nuclear retention of Stat molecules.

Most striking is the complete loss of nucleocytoplasmic shuttling during nuclear accumulation proposing that the mutants bear a dephosphorylation defect *in-vivo.* Tyrosine dephosphorylation is localized within the N-domain representing the crucial step in the control of nuclear export. Both, the dephosphorylation defect as well as the tetramerization deficiency affect the nuclear accumulation of the mutants concluding that the interfaces for dimer-phosphatase and dimer-dimer interactions are overlapping.

In summary, the mutated Stats are still functional transcription factors that retain cytokine responsiveness. The interface centered around the critical hydrophobic residue, mainly represented by phenylalanine, fulfills distinct functions that are subjected to control by the tyrosine phosphorylation and DNA binding status of the Stat molecules. Due to the mutation, the gene induction depends on promoter strength whereby either an increase or a decrease of the transcription activity could be recognized.

In another embodiment of the invention, the wild type of Stat2, Stat3, Stat5 or Stat6 is mutated. The Stat molecule is mutated in at least one conserved hydrophobic amino acid on the surface of the N-domain and characterized in that the Stat molecule is derived from the wild type of Stat2, Stat3, Stat5 or Stat6.
The Stat family of transcription factors comprise seven mammalian proteins with known homologues in all multicellular animal phyla. Stat proteins are activated by a wide range of different cytokines such as interleukins and prolactin, as well as growth factors. Accordingly, gene ablation experiments uncovered mammary gland defects, growth abnormalities and an absent interleukin response underlining the significance of regulating the Stat activity as tool for gene induction. In particular, disturbances in the signaling pathways were figured out as cancer inducing parameter. Among the Stat molecules, only Stat2, Stat3, Stat5 and Stat6 were found in the nuclei of cutaneous T cell lymphoma cell lines suggesting a selective malfunction of some transcription factors.

In another preferred embodiment of the invention, the amino acids of the N-domain in position 81 and/or position 82 of Stat5 or in equivalent positions of the homologous proteins are mutated.
Stat5a and Stat5b are two highly related proteins with a sequence identity of ~96%. Possible mutations include the substitution, deletion or modification of the amino acid. They may occur in position 81 and/or position 82 of Stat5 or in equivalent positions of homologous proteins. Homologous proteins are members of the aforementioned Stat family, preferably Stat2, Stat3, Stat5 or Stat6. Although they display a great sequence similarity, minor shifts of distinct residues within the primary structure are present. With regard to higher structure levels (secondary and/or tertiary structure), the shifted positions are referred to as equivalent. For example, the positions 77 and 78 in Stat 3 and Stat6 as well as the positions 81 and 82 in Stat2 and Stat5 are considered as equivalent.
In consequence of their mutation, the residues in position 81 and/or position 82 of Stat5 or equivalent in homologous proteins cannot maintain N-domain interactions in the full-length Stat molecule resulting in the linked phenomena of tetramerization and dephosphorylation-deficiency, and their consequences on gene induction in a promoter-specific manner.

In another preferred embodiment of the invention, the amino acid in position 81 of Stat5 or in the equivalent position of the homologous proteins is substituted by alanine.
Due to its neutral properties originated from the short side chain of a single methyl group, alanine is especially suited for mutagenesis studies. Within the Stat family, different residues in an equivalent position are present. These differences are preferably of equivalent matter by changing a hydrophobic amino acid by another hydrophobic one, for example. The hydrophobic residues in the equivalent positions 77 and 81 comprise the following amino acids within those Stat molecules preferred in the meaning of the invention: leucine in position 81 of Stat2, valine in position 77 of Stat3, phenylalanine in position 81 of Stat5 and isoleucine in position 77 of Stat6.

The amino acid sequence of the mutants termed Stat5aF81A or Stat5bF81A, respectively, are given below:

Human Stat5aF81A (1-710):
magwiqaqql qgdalrqmqv lygqhfpiev rhylaqwies qpwdaidldn
pqdraqatql leglvqelqk kaehqvgedg allkiklghy atqlqktydr
cplelvrcir hilyneqrlv reanncsspa gilvdamsqk hlqinqtfee
lrlvtqdten elkklqqtqe yfiiqyqesl riqaqfaqla qlspqerlsr
etalqqkqvs leawlqreaq tlqqyrvela ekhqktlqll rkqqtiildd
eliqwkrrqq lagnggppeg sldvlqswce klaeiiwqnr qqirraehlc
qqlpipgpve emlaevnati tdiisalvts tfiiekqppq vlktqtkfaa
tvrllvggkl nvhmnppqvk atiiseqqak sllknentrn ecsgeilnnc
cvmeyhqatg tlsahfrnms lkrikradrr gaesvteekf tvlfesqfsv
gsnelvfqvk tlslpvvviv hgsqdhnata tvlwdnafae pgrvpfavpd
kvlwpqlcea lnmkfkaevq snrgltkenl vflaqklfnn ssshledysg
lsvswsqfnr enlpgwnytf wqwfdgvmev lkkhhkphwn dgailgfvnk
qqahdllink pdgtfllrfs dseiggitia wkfdspernl wnlkpfttrd
fsirsladrl gdlsyliyvf pdrpkdevfs kyytpvlaka vdgyvkpqik

Human Stat5bF81A (1-715):
mavwiqaqql qgealhqmqa lygqhfpiev rhylsqwies qawdsvdldn
pqenikatql leglvqelqk kaehqvgedg allkiklghy atqlqntydr
cpmelvrcir hilyneqrlv reanngsspa gsladamsqk hlqinqtfee

lrlvtqdten elkklqqtqe yfiiqyqesl riqaqfgpla qlspqerlsr
etalqqkqvs leawlqreaq tlqqyrvela ekhqktlqll rkqqtiildd
eliqwkrrqq lagnggppeg sldvlqswce klaeiiwqnr qqirraehlc
qqlpipgpve emlaevnati tdiisalvts tfiiekqppq vlktqtkfaa
tvrllvggkl nvhmnppqvk atiiseqqak sllknentrn dysgeilnnc
cvmeyhqatg tlsahfrnms lkrikrsdrr gaesvteekf tilfesqfsv
ggnelvfqvk tlslpvvviv hgsqdnnata tvlwdnafae pgrvpfavpd
kvlwpqlcea lnmkfkaevq snrgltkenl vflaqklfnn ssshledysg
lsvswsqfnr enlpgrnytf wqwfdgvmev lkkhlkphwn dgailgfvnk
qqahdllink pdgtfllrfs dseiggitia wkfdsqermf wnlmpfttrd
fsirsladrl gdlnyliyvf pdrpkdevys kyytpvpces atakavdgyv
kpqikqvvpe fvnas

This invention is based on a mutation which is bold.

In another preferred embodiment of the invention, the amino acid in position 82 of Stat5 or in the equivalent position of homologous proteins is substituted by serine. Preferably, the amino acid in position 82 of Stat5 or in the equivalent position of homologous proteins is leucine.

The mutants Stat5aF81A and Stat5bF81A of this invention display a couple of novel features which are contrary to the wild type phenotype.
The mutants of this invention are tetramerization-deficient causing a promoter-dependent decrease in the strength of DNA binding. Their ability to bind tandemly linked GAS motifs in a cooperative manner is completely lost in comparison with the wild type. Contrary, the binding to a single optimal GAS site is similar to the wild type indicating a DNA recognition as dimer only. Surprisingly, this dimerization is contradictory to the observed occurance as monomer by Chen *et al*., Protein Science 12, 361-365 (2003). In consequence of the usage of natural promoter fragments, the reduced DNA binding is accompanied by a reduced transcription activity as well. Since natural promoters often harbor a weak single canonical GAS site, Stat oligomerization is required for high transcriptional efficiency. This ability to form tetramers is tested by EMSAs (electrophoretic mobility shift assays).

Whereas any impaired DNA binding normally results in shortened nuclear retention, the nuclear accumulation of the Stat mutants is uncoupled from the DNA binding capacity. The mutated phenotype displays a Stat accumulation which is 20 to 300%, preferably 35 to 200% and more preferably 50 to 100% prolonged compared to the wild type. The addition of vanadate which inhibits tyrosine phosphatase activity even results in increased nuclear concentrations of mutated Stat dimers in a range from 10 to 90%, preferably 30 to 70% and more preferably from 45 to 55%. Furthermore, due to the tetramerization defect the mutants form oligomers less frequently and thus maintain a high intranuclear mobility which is equivalent to the wild type.

Stat5aF81A and Stat5bF81A show prolonged tyrosine phosphorylation and insensitivity towards staurosporine, which is indicative of a reduced dephosphorylation rate. Interestingly, in the IL-2 receptor-reconstituted 293T system the kinetics of dephosphorylation of wild type Stat5 proteins is longer (2 to 8 h, preferably 2 to 6 h and more preferably 2 to 4 h) than that observed in lymphoid cells, where it occurs within 0.3 to 2 h, preferably 0.5 to 2 h and more preferably 1 to 2 h. Nevertheless, mutation of residue 81 also prolongs tyrosine phosphorylation even further, as the phosphotyrosine signal now remains detectable for more than 10 h, preferably 8 h and more preferably 6 h.

In the presence of proteasome inhibitors, the sensitivity of the mutants to hyperphoshorylation caused by a constituent of the IL-2 pathway is increased again to 20 to 300%, preferably 35 to 200% and more preferably 50 to 100% if compared to the wild type.

Finally, the N-domain point mutation has an impact on the gene transcription in a promoter-specific manner. Reporter genes containing optimal GAS sites are transactivated as good as or even better up to 300%, preferably up to 200% and more preferably up to 100% by the Stat mutants than by the respective wild type counterpart. The effective binding to such sites is less dependent upon cooperativity since the multimerization of optimal sites mimics oligomerization. In contrast, when natural promoter fragments are used, transactivation is lower up to 100%, preferably up to 75% and more preferably up to 50% with the mutants in comparison to the wild type Stat molecule. Here, Stat oligomerization acts as requirement of the subsequent DNA binding ability and an adequate gene induction.

In another preferred embodiment of this invention, the mutated Stat molecules are dimerized.
Stat dimers are the basic scaffold for subsequent DNA binding. Based on the dimerization, Stat1 and Stat3 to Stat6 are able to form higher order complexes (dimer-dimer or higher) on any DNA promoter which does not display an optimal consensus sequence. However, this inherent feature of higher oligomerization is abrogated in the described mutants.

The present invention also relates to a fusion protein comprising the mutated Stat molecule and at least one marker structure.
A "fusion protein" is a protein which consists of amino acid sequences derived from at least two different sources. In context with fusion proteins, a "heterologous" amino acid sequence is a sequence derived from a source other than the source of the other components of the fusion protein.

In the meaning of the invention, heterologous "marker structures" are understood to be proteins which are coupled to the Stat mutants, enabling the detection of them by means of various biological, chemical and/or physical methods. Advantageously, it is possible to localize proteins under investigation in a living cell, especially in a host cell. Furthermore, it is possible e. g. to monitor the migration of proteins in real-time investigations.

A reporter gene encodes such a detectable marker protein. The reporter gene according to the invention either can be the overall DNA construct of the protein to be detected. However, it may also be not more than that DNA portion of the protein which has detectable activity. Consequently, any DNA structure encoding a region detectable by means of various detection methods may serve as reporter gene. Preferred in this context are expression constructs encoding proteins which can be detected easily and with high sensitivity.

For example, the inventive marker structure in the fusion protein can be chloramphenicol acetyl transferase (CAT). In particular, CAT catalyzes the transfer of an acetyl residue from acetyl-CoA to chloramphenicol. Among other things, CAT has the advantage of being stable in a cell, with a half-life of about 50 h. This is particularly advantageous where e. g. yields of reporter gene as high as possible are to be achieved. Advantageously, CAT has a good signal/background ratio.

Another advantageous marker structure in the meaning of the invention is luciferase. Luciferase advantageously has a high sensitivity and, owing to a relatively short half-life of about three hours, it is well-suited for induction studies. Particularly advantageous is a marker structure including two luciferases with distinct properties, one luciferase, e. g. from *Phottinus pyralis,* detecting the activity of the own promoter, while the co-transfected luciferase, e. g. from *Renilla reniformis,* is under control of the constitutive promoter and thus can be used advantageously for standardization purposes.

Further examples of marker structures in the meaning of the invention are β-galactosidase, human growth hormone and secreted alkaline phosphatase. Advantageously, the marker structures of the invention can also be used as reporter genes in qualitative detection. In qualitative detection, it is possible e. g. to determine whether a selected cell has been transfected or not, or, it is possible to determine in the entire organism, in which type of tissue a promoter is active. For example, reporter genes for qualitative detection are the genes for luciferase and β-galactosidase.

In another preferred embodiment of the invention, the marker structure is green fluorescent protein (GFP), glutathione S-transferase (GST) and/or a fragment thereof. The green fluorescent protein advantageously has low cytotoxic activity or none at all. Advantageously, the absorption and emission maxima of green fluorescent protein are similar to that of fluorescein, so that e. g. a GFP-positive cell can be detected using the same methods as with cells stained with fluorescein, i. e., under UV light, under a fluorescence microscope, or in a cell sorter (FACS), for example.

Furthermore, preferred marker proteins which can be fused with the Stat mutants are GST as well as other fluorescent proteins such as the red or the yellow protein, as well as the maltose-binding protein (MBP), and others.

In another advantageous embodiment, the fusion protein contains the marker structure at the N-terminus and/or the C-terminus. The arrangement will depend on the maintenance of the three-dimensional structure and the functional activity of both Stat mutant and marker structure. The integration within the Stat mutant may disrupt its intermolecular order, however it should not be excluded.

The invention also relates to a nucleic acid sequence which encodes the Stat mutant or the fusion protein.
The term "nucleic acid sequence" refers to a natural or synthetic polymer of single- or double-stranded DNA or RNA alternatively including synthetic, non-natural or modified nucleotides which could be incorporated in DNA or RNA polymers. Each nucleotide consists of a sugar moiety, a phosphate moiety, and either a purine or pyrimidine residue.

The term "nucleic acid sequence which encodes" refers to that part of a gene which enciphers a protein, a polypeptide or a part thereof. The regulatory sequences and/or elements controlling the initiation or termination of transcription are excluded. The coding sequence and/or the regulatory element can normally be found in cells, in which case it is referred to as "autologous" or "endogenic", or cannot be situated in cells, in which case it is referred to as "heterologous".

"Gene" denotes a DNA sequence encoding a specific protein, and regulatory elements controlling the expression of said DNA sequence.
A heterologous gene may also be composed of autologous elements arranged in an order and/or orientation which is normally not found in that cell the gene is transferred into. A heterologous gene can be derived completely or partially from any source known in the art, including a bacterial or viral genome or episome, eukaryotic nuclear or plasmid DNA, cDNA, or chemically synthesized DNA. The structural gene may form a continuous coding region, or may comprise one or more introns bordered by suitable splice junctions. The structural gene can consist of segments derived from various naturally occurring or synthetic sources.

The present invention also relates to a vector comprising the above-described nucleic acid sequence, particularly to a bacterial vector such as a plasmid, or a virus.

The term "vector" denotes a recombinant DNA construct which can be a plasmid, a virus, or an autonomously replicating sequence, a phage, or a nucleotide sequence, which is linear or circular, consists of single- or double-stranded DNA or RNA, wherein a number of nucleotide sequences are linked or recombined to form a unique construction, and which is capable of introducing a promoter fragment and a DNA sequence of a selected gene product in sense or antisense orientation into a cell, together with suitable non-translated 3' sequences.

"Plasmids" are genetic elements which are stable inherited without being part of the chromosome of their host cell. They may comprise DNA or RNA, and they can be both linear and circular. Plasmids encode molecules ensuring their replication and stable inheritance during cell replication, and they are capable of encoding products of considerable importance for medicine, agriculture and environment. For example, they encode toxins which massively increase the virulence of pathogenic bacteria. They are also capable of encoding genes imparting resistance to antibiotics. In molecular biology, plasmids are generally used as vectors to clone and express recombinant genes. According to the standard nomenclatural rules familiar to those skilled in the art, plasmids are generally described using the small letter p preceded or followed by capital letters and/or numerals. The starting plasmids disclosed in the present specification either are commercially available, accessible to the public, or can be constructed from available plasmids by routine use of well-known, published methods. Many plasmids and other cloning and expression vectors which can be used according to the invention are well-known and easily available to the skilled artisan. Furthermore, a person skilled in the art can easily construct any number of other plasmids suitable for the use in this invention. The properties, construction and use of such plasmids, as well as other vectors, are readily accessible to those skilled in the art from the present disclosure.

Moreover, the present invention relates to host cells transfected with a vector of the invention, particularly to prokaryotic or eukaryotic cells. The present invention also relates to cell cultures, tissues, organs and the like, which comprise cells including an above-described plasmid or vector.
The term "host cell" relates to a cell which has been genetically modified by the transfer of a chimeric, heterologous or autologous nucleic acid sequence or derivatives thereof still including said sequence. These cells are also referred to as "transgenic cells". Where an autologous nucleic acid sequence is transferred, the number of copies of this sequence in the host cell is higher than that of the naturally occurring sequences.

Object of the present invention is also the use of the Stat mutants of the invention, their dimers, the fusion protein of the invention, the nucleic acid sequence of the invention, the vector of the invention or the host cell of the invention for expression of a target protein, for investigating effects caused by different gene induction levels, for screening of active substances which modify the transcription activity of target genes, for identifying binding partners or for the production of a drug for the diagnosis and/or treatment of diseases associated with abnormal Stat induction of target genes.

Binding assays are usually performed to detect protein-protein interactions. A well-established system represents the two-hybrid system, preferably a yeast two-hybrid system. The latter exploits the modular nature of many transcription factors which are typically composed of a DNA-binding domain (DBD) and an activation domain (AD). The original transcription factor is split and two types of hybrids are created: the first one is a DBD-bait fusion, in which the bait represents a protein of interest or a fragment thereof, and the second hybrid comprises an AD-prey fusion, in which the prey represents a protein library to be investigated. Hyperactive STAT proteins may be exploited for interaction trap assays such as the yeast two-hybrid system. In these assays a probe or "bait" protein fused to a heterologous DNA-binding protein domain reacts with components of a conditionally expressed library comprising fusion proteins with a portable transcriptional activation domain. The function of STAT proteins as constitutive shuttling molecules may facilitate the search for interactions between the bait and the fusion proteins harboring a transactivation domain.
Furthermore, in the meaning of the invention, any of the Stat mutants could act as bait which is fused to the LexA DBD, for example, whereas the protein library could be merged with the Gal4 AD domain. Stat mutant-LexA DBD fusion proteins encoded in a first vector and library-Gal4 AD fusion proteins encoded in a second vector are transformed into two yeast strains which are cultured under appropriate conditions and over an appropriate period. Subsequently, the yeast strains are mated, grown on plates and examined for LacZ expression. The identified binding partners may elucidate the control of signaling pathways and/or different gene induction levels, for instance by cooperative binding. In addition, they could be useful as markers for pathogenic phenotypes and/or as activators or inhibitors of Stat molecules in order to modify the transcription activity of target genes.

The present invention also relates to a method for the expression of a target protein including the addition of the mutated Stat monomer of the invention, their dimers or the fusion protein of the invention to an *in-vitro* system containing genes under Stat control. The *in-vifro* system preferably represents a cell and more preferably a cell-free system. Furthermore, the method for the expression of a target protein includes the nucleic acid sequence of the invention preferably inserted into the vector of the invention which is preferably transformed or transfected into the host cell of the invention.

The Stat mutants exert an influence on gene activation in a promoter-specific manner. While the transcription yield is strongly enhanced if activated on optimal GAS sites, the opposite phenomenon appears on natural promoter sequences. Thus, either an overexpression or an underexpression of a target protein can be realized compared to the matching gene induction by the Stat wild type.

A suitable vector containing a promoter of choice followed by the DNA sequence of the Stat mutant is transformed into an appropriate cell culture. Depending on the cell culture, a phosphorylation system could be included for Stat activation. Since cell-inherent genes are usually induced on weak promoter sites, their yield would be additionally dropped by using Stat mutants. In order to reverse that expression level, the promoter sequence should be mutated as well. Alternatively, a construct of promoter harboring at least a single optimal GAS sequence and a target gene of choice is preferably inserted into a second vector. High protein amounts can be advantageously obtained in this way.

In addition to the cellular expression, the cell-free system is applicable.
Cell-free systems involve coupled transcription and translation reactions *in-vitro* which are based on a cell lysate (generally called S30). The lysate is pre-produced by means of cell disruption and subsequent centrifugation at 30.000 g. The advantages of the cell-free protein biosynthesis include, *inter alia,* the solely dependence of protein expression on the template, high yields as well as the possibilities to produce cell-toxic proteins and to incorporate unusual amino acids into the protein.
Two vectors which are generated to harbor the Stat insert and the target gene as described in the cellular *in-vitro* section are introduced to the cell-free system. The encoded proteins are expressed and the tyrosine phosphorylation of the Stat mutant is performed with EGF receptor or supplemented Jak.

Instead of the simultaneous expression of Stat mutant and target protein in the cell-free system, the addition of a mutated Stat monomer, a dimerized Stat molecule or a fusion protein separately expressed in advance is imaginable.
The term "expression" used in the present specification is intended to describe the transcription and translation of the sequence of the gene product. Where the gene product is a protein, the expression involves initial transcription of a DNA chain encoding the sequence of a gene product, to form a complementary RNA, frequently a mRNA, followed by the translation of the mRNA, to form the above-mentioned gene product. However, expression also comprises transcription of a DNA inserted in antisense direction with respect to its regulatory elements. An expression which proceeds in a constitutive fashion and is possibly further enhanced by an externally controlled promoter fragment - to produce multiple mRNA copies and large amounts of the selected gene product - may also involve overproduction of a gene product.

For expression of recombinant Stat proteins the wild type Stat cDNA sequence preferably cloned into a baculovirus transfer vector is mutated by site-directed mutagenesis at the appropriate residues which is confirmed by DNA sequence analysis. Tyrosine phosphorylation is checked by Western blot analysis using phosphotyrosine-specific antibodies.

The present invention also relates to a method for investigating effects caused by different gene induction levels including the addition of the mutated Stat monomer of the invention, their dimers or the fusion protein of the invention to an *in-vitro* system containing genes under Stat control. In the meaning of the invention, such effects are understood to be any influence on transcription activity, resulting in an increase or drop in activity, which can be determined by means of biological, chemical or physical measuring methods. The *in-vitro* system preferably represents a cell and more preferably a cell-free system. Furthermore, the method for investigating effects caused by different gene induction levels includes the nucleic acid sequence of the invention preferably inserted into the vector of the invention which is preferably transformed or transfected into the host cell of the invention.

The Stat mutation of the tetramerization interface affects the transcription in a promoter-specific manner. Depending on the overexpression or the underexpression of selected target proteins, biochemical effects on the living cell will appear. They may concern the entire range of biochemical life processes, like metabolic rate, cell growth, proliferation, aging etc. The up- or down-regulation of genes is tightly controlled. The resulting observations are very helpful to reveal unknown functions of genes.
The general experimental procedure is identical to that already described for the method for expression performed in cells. The monitoring of the effects depends on the target gene to be investigated. A couple of methods to detect a gene product is mentioned within the description of the next embodiment concerning the screening of active substances.
A "detectable gene product" is a nucleotide sequence or amino acid sequence which can be detected by means of an appropriate test depending on the gene product itself. Preferably, the expression of a detectable gene product provides the cell with a feature allowing easy selection of that cell among other cells not expressing the detectable gene product.

The present invention also relates to a method for the screening of active substances which modify the transcription activity of target genes including the addition of the mutated Stat monomer of the invention, their dimers or the fusion protein of the invention to an *in-vitro* system containing genes under Stat control. In the meaning of the invention, such modifying is understood to be any influence on transcription activity, resulting in an increase or drop in activity, which can be determined by means of biological, chemical or physical measuring methods. The *in-vitro* system preferably represents a cell and more preferably a cell-free system. Furthermore, the screening method includes the nucleic acid sequence of the invention preferably inserted into the vector of the invention which is preferably transformed or transfected into the host cell of the invention.

Generally, a suitable expression vector containing a promoter of choice followed by the DNA sequence of the Stat mutant is transformed into an appropriate cell culture firstly.
A target gene which is cell-inherent can be chosen thereby exclusively investigating the effect of an active substance on the interaction between Stat mutant and natural promoters. Alternatively, the construct of promoter and reporter gene of choice is preferably inserted into a second vector choosing a weak natural promoter (e. g. PRRIII or ICAM-1) for the screening of transcription activators or a strong synthetic promoter (e. g. the PRRIII derivative M10 or Ly6E) for the opposite selection of transcription inhibitors. For example, the treatment of human malignancies requires agents that reduce Stat levels and/or Stat activity, along with the promoter-binding capability. Besides, a fusion protein construct can be used to recognize Stat relocations. Those skilled in the art can easily produce these constructs by well-known recombination and cloning techniques.

For screening, a couple of methods are to be distinguished by the quantitative determination either of the product of transcription or that of translation.
On the level of transcription, the synthesized RNA can be detected via DNA by semiquantitative RT-PCR. Cells stable expressing any mutant are cultivated and spread on a microtiter plate containing a substance library. Cells are treated with cytokines, like the interferons IFNα and IFNγ or interieukins, to induce corresponding responsive genes. They are kept interacting for a certain time before the RNA is extracted and reverse transcribed followed by PCR. The PCR products can be analyzed either directly or by gel electrophoresis and quantified by scoring the intensities of ethidium bromide staining. The results are finally compared to the untreated cell sample thus revealing the mode and direction of changes in mRNA yield.
Likewise, the cell-free system already described can be treated with a library of substances followed by the detection of the target mRNA.

The protein can be translated either in cells or in the cell-free system.
In the first one, eukaryotic cells are transfected with the plasmid constructs as described above. After a certain period of adaptation the cells are spread on a microtiter plate containing a library of substances to be screened. The Stat transport to the nucleus is induced by administering the appropriate cytokines leading to nuclear accumulation firstly. Secondly, changes in the expression level of target genes may occur due to association of library substances with Stat mutants.

In the context of the present invention, the term "association" - without being limited thereto - relates to any type of interaction between the Stat molecule and the target, particularly covalent or non-covalent binding or association, such as a covalent bond, hydrophobic/hydrophilic interactions, van der Waals forces, ion pairs, ligand-receptor interactions, interactions between epitope and antibody binding site, nucleotide base pairing, and the like. Such association may also encompass the presence of other molecules such as peptides, proteins or other nucleotide sequences.
As an example in the context of the invention, the changed yield of a reporter gene product as marker for transcription activity is monitored via firefly luminescence in the luciferase assay system.

For the detection of cell-inherent gene products, an immunoassay is applicable.
"Immunoassay" denotes a test wherein an antibody is used for specific binding an analyte. The immunoassay is characterized in that specific binding properties of a particular antibody are utilized in the isolation, purposeful testing and/or quantitative determination of the analyte. Those skilled in the art can easily produce antibodies directed against the target protein to be monitored and fragments thereof.
"Antibody" denotes a polypeptide essentially encoded by an immunoglobulin gene or fragments thereof specifically binding and recognizing an analyte (antigen). Known immunoglobulin genes include the kappa, lambda, alpha, gamma, delta, epsilon, and mu genes for the constant region, as well as the innumerable genes for the variable immunoglobulin region. For example, antibodies are present as intact immunoglobulins or as a number of well-characterized fragments produced by cleavage using various peptidases.

An antibody binds specifically to a protein, e. g. another biological structure, or exhibits specific immunoreactivity in this way, if the antibody assumes its function in a binding reaction in the presence of a heterogeneous population of proteins and other biological substances, which reaction allows a decision to be made whether the protein or another biological structure is present. Under the established conditions of an immunoassay, the antibodies mentioned preferably bind to a specific protein, whereas no significant binding to other proteins present in the sample takes place. Specific binding to a protein under such conditions requires an antibody which has been selected according to its specificity for a particular protein. Various variants of immunoassays can be used to select antibodies having specific immunoreactivity with a particular protein. A description of immunoassay variants and conditions which can be used to determine a specific immunoreactivity is presented in Harlow & Lane, Antibodies; A Laboratory Manual (1988), Cold Spring Harbor Publications, New York.

In addition to the cellular gene expression and substance screening, the application of the cell-free system is imaginable. Two vectors which are generated to harbor the Stat insert and the target gene as described in the cellular *in-vitro* section are introduced to the cell-free system which is subsequently spread on a microtiter plate containing a library of substances. The encoded proteins are expressed and the tyrosine phosphorylation of the Stat mutant is performed with EGF receptor or supplemented Jak.
Instead of the simultaneous expression of Stat mutant and target protein in the cell-free system, the addition of a mutated Stat monomer, a dimerized Stat or a fusion protein separately expressed in advance is possible as already described above.

The present invention also relates to a method for the production of a drug for the diagnosis and/or treatment of diseases associated with abnormal Stat induction of target genes including the detection of the mutated Stat monomer of the invention, their dimers or the fusion protein of the invention in a cell. Furthermore, the method for the production of a drug includes the nucleic acid sequence of the invention preferably inserted into the vector of the invention which is preferably transformed or transfected into the host cell of the invention.

According to the invention, a "disease associated with abnormal Stat induction of target genes" is arthritis, carcinogenesis, nephritis, proteinuria, dermatitis, diabetes/obesity, acute and chronic rejection of allogeneic organ transplants, neuron degeneration, Parkinson's disease, septic shock, endotoxemia, hypersensitivity, uveitis, wound closure, and others. The abnormal Stat induction of target genes also plays an important role in the regulation of the immune system, in inflammation and effector mechanisms of cellular and humoral immunity, septic shock, inflammation of sensory organs, like the eyes, in the regulation of transcription, cell cycles and cell growth, acute respiratory insufficiency, physiological stress, numerous cardiovascular diseases and other disorders.

In the context with the present invention, the term "treatment" refers to the prophylactic and/or therapeutic effect of a drug.
Dysfunctions of signaling pathways are e. g. reflected by inappropriate activation and accumulation of Stat molecules. Therefore, agents that inhibit Stat functions by a decrease of Stat levels and/or binding specifity to target DNA sequences would represent a promising approach which may have the potential to be more selective and less toxic than current anti-cancer treatments. The phenotype of the Stat mutants this invention is based on corresponds with the mentioned requirements. The interactions of the alternate N-domain interface of the mutants which do not mediate oligomerization and block dephosphorylation are probably accompanied by a three-dimensional structural rearrangement. Hence, the application of high affinity molecules which are able to discriminate between the wild type structure and the mutated one is suggested for diagnosis. These high affinity molecules involve antibodies already described or RNA aptamers. The latter are identified by an *in-vitro* selection procedure - the so-called SELEX process (systematic evolution of ligands by exponential enrichment). Since RNA is very susceptible to nucleolytic degradation in biological solutions, aptamers are chemically modified using phosphorothioates, locked nucleic acids or Spiegelmers™. Because of their high affinity for a broad spectrum of structural targets, aptamers act very similar to antibodies. Both, antibodies and aptamers carry functional coumpounds which are detectable to determine their binding quantity to the Stat target. As example, the fluorescence labelling is mentioned.

Referring to a desired reduction of Stat activity for the treatment of diseases caused by a hyperactivation, the wild type Stat molecule should be replaced by the Stat mutant. This transfer may happen by means of a gene therapy. The most common method involves the introduction of genetic material into a gene shuttle, for instance retro viruses. The replacement of the Stat gene in certain cell types can also be performed *in-vitro* and the modified cells are subsequently re-injected into the organism. The recombination of the vehicle genome or the cell genome, respectively, with the nucleic acid sequence encoding the Stat mutant follows standard techniques, whereas targeting and the stable incorporation of the new gene represent current research topics.

The present invention also relates to a method for the importing and targeting of substances into the nucleus which includes the vector encoding the proteins of the invention. The vector is preferably transformed or transfected into the host cell of the invention.
These substances preferably represent peptides or proteins which can be attached to any terminus of the Stat molecule and simultaneously expressed as fusion construct. Firstly, the peptides or proteins should be able to interact specifically with physiologically interesting endogenous compounds of the nucleus or exogenous compounds expressed by co-transfection. Secondly, these imported substances have to display a higher target affinity in comparison to the carrier Stat molecule. The latter fact is essential for their preferred successful association to the target. Inhibitors and activators of transcription which bind to DNA or enzymes are suited molecules to be transferred into the nucleus.

Eukaryotic cells are transfected *in-vitro* with the vector encoding the Stat mutant in combination with an active peptide structure. Those skilled in the art can easily produce these constructs by well-known recombination and cloning techniques. Alternatively to the single Stat mutant, the fusion protein containing a marker structure can be used to pursuit Stat relocations during import and within the nucleus. The transport to the nucleus is induced by administering interferon or interleukin, respectively, leading to nuclear accumulation. The effects of the imported substance on the cell metabolism are detected by an appropriate method depending on the gene product.

Furthermore, the construct of Stat mutant and active substance may be introduced *in-vivo* by using a vehicle like a virus. In this case, the procedure is to be considered as gene therapy.

All statements concerning the use and the methods for several purposes also include a Stat molecule derived from wild type Stat1, which is mutated in at least one conserved hydrophobic amino acid on the surface of the N-domain, preferably in the amino acids in position 77 and/or position 78. The mutation in position 77 may involve a substitution by alanine, and the mutation in position 78 may involve a substitution by serine. There are two slicing variants alpha and beta differing in the terminal amino acid sequence from 713 to 750 which is absent in the beta isoform. The mutants of the following sequences are termed Stat1 αF77A and Stat1 βF77A.

Human Stat1αF77A (1-750):
msqwyelqql dskfleqvhq lyddsfpmei rqylaqwlek qdwehaandv
sfatirfhdl lsqlddqysr fslennallq hnirkskrnl qdnfqedpiq
msmiiysclk eerkilenaq rfnqaqsgni qstvmldkqk eldskvrnvk
dkvmciehei ksledlqdey dfkcktlqnr ehetngvaks dqkqeqlllk
kmylmldnkr kevvhkiiel lnvteltqna lindelvewk rrqqsacigg
ppnacldqlq nwftivaesl qqvrqqlkkl eeleqkytye hdpitknkqv
lwdrtfslfq qliqssfvve rqpcmpthpq rplvlktgvq ftvklrllvk
lqelnynlkv kvlfdkdvne rntvkgfrkf nilgthtkvm nmeestngsl
aaefrhlqlk eqknagtrtn egplivteel hslsfetqlc qpglvidlet
tslpvvvisn vsqlpsgwas ilwynmlvae prnlsffltp pcarwaqlse
vlswqfssvt krglnvdqln mlgekllgpn aspdglipwt rfckenindk
nfpfwlwies ilelikkhll plwndgcimg fiskereral lkdqqpgtfl
lrfsessreg aitftwvers qnggepdfha vepytkkels avtfpdiirn
ykvmaaenip enplkylypn idkdhafgky ysrpkeapep meldgpkgtg
yiktelisvs evhpsrlqtt dnllpmspee fdevsrivgs vefdsmmntv

Human Stat1βF77A (1-712):
msqwyelqql dskfleqvhq lyddsfpmei rqylaqwlek qdwehaandv
sfatirfhdl lsqlddqysr fslennallq hnirkskrnl qdnfqedpiq
msmiiysclk eerkilenaq rfnqaqsgni qstvmldkqk eldskvrnvk

dkvmciehei ksledlqdey dfkcktlqnr ehetngvaks dqkqeqlllk
kmylmldnkr kevvhkiiel lnvteltqna lindelvewk rrqqsacigg
ppnacldqlq nwftivaesl qqvrqqlkkl eeleqkytye hdpitknkqv
lwdrtfslfq qliqssfvve rqpcmpthpq rplvlktgvq ftvklrllvk
lqelnynlkv kvlfdkdvne rntvkgfrkf nilgthtkvm nmeestngsl
aaefrhlqlk eqknagtrtn egplivteel hslsfetqlc qpglvidlet
tslpvvvisn vsqlpsgwas ilwynmlvae prnlsffltp pcarwaqlse
vlswqfssvt krglnvdqln mlgekllgpn aspdglipwt rfckenindk
nfpfwlwies ilelikkhll plwndgcimg fiskereral lkdqqpgtfl
lrfsessreg aitftwvers qnggepdfha vepytkkels avtfpdiirn
ykvmaaenip enplkylypn idkdhafgky ysrpkeapep meldgpkgtg
yiktelisvs ev

The mutated amino acid is bold.

All statements concerning the methods for several purposes also include the addition or detection of the dimerized Stat1 mutant, which is mutated as described above, or of a fusion protein including the Stat1 mutant and at least one marker structure. Furthermore, these methods include the nucleic acid sequence which encodes the Stat1 mutant or the fusion protein preferably inserted into a vector which is preferably transformed or transfected into the host cell.

Their integration into any special method mentioned proceeds in accordance to the previous description. The methods are performed identically to those already described for other Stat derivatives.

The present invention also relates to a kit comprising the amino acid sequence or the nucleic acid sequence of the Stat mutants of the invention, their dimers or fusion proteins, and/or synthetic analogues, modifications and pharmacologically active fragments thereof and an information about the using of the kit.

The kits to be built-up are either based on the genotype or the phenotype of the Stat mutant. They can also include parts of the amino acid sequence or the nucleic acid sequence originated from the Stat mutant, a vector harboring the nucleic acid sequence or a host cell containing the vector. The kits should be preferably applied for any of the methods described above.

The following examples are provided by way of illustration and not by way of limitation. Within the examples, standard reagents and buffers that are free from contaminating activities (whenever practical) are used. It is preferred to exercise care to avoid ribonucleases and PCR product contamination.

### 1. Example

The plasmid expressing full-length human STAT1 (amino acids 1-746) fused to the N-terminus of GFP (pSTAT1-GFP) was described previously (Begitt *et al.,* Cell Biol. 97, 10418-10423 (2000)). Stat expression constructs were generated by cloning the Stat1 full-length coding region in the *Bam*HI and *Not*I sites of the vector pcDNA3 (Invitrogen). Those skilled in the art can easily produce these constructs by well-known recombination and cloning techniques.
RT-PCR analysis of endogenous IFNα- and IFNγ-responsive genes was performed with U3A cells stably expressing Stat1 wild type or Stat1 F77A mutant. Cells were grown for 15 h in Dutbecco's modified Eagle's medium with reduced serum (1%), followed by the addition of serum to 10% and treatment with or without IFNα or IFNγ for 4 h. RNA was extracted and reverse transcribed followed by PCR. The following primer pairs were used: IRF-1 (GenBank™ accession number NM_002198) 5'-ATGAGACCCTGGCTAGAG-3' and 5'-AAGCATCCGGTACACTCG-3'; LMP-2 (GenBank™ accession number U01025) 5'-GG-GGGCGTTGTGATGGGTTCTGATTC-3' and 5'-TCGGGAGACATGCCTGGCTTATAT-3'; ISG-15 (GenBank™ accession number NM_005101) 5'-GGCGGGCAACGAATTCCAGGTGTC-3' and 5'-CCCCGCAGGCGCAGATTCATGA-3'; ISG-56k (GenBank™ accession number M24594) 5'-AGGGCAGAACAGAGAAAAGCTAGACAA-3' and 5'-CCGCTCATAGTACTCCAGGGCTTCATTC-3'. The ethidium bromide-stained PCR products were quantified. Absolute intensities were scored in arbitrary units, and the ratio between induced and uninduced signal was depicted for each gene.

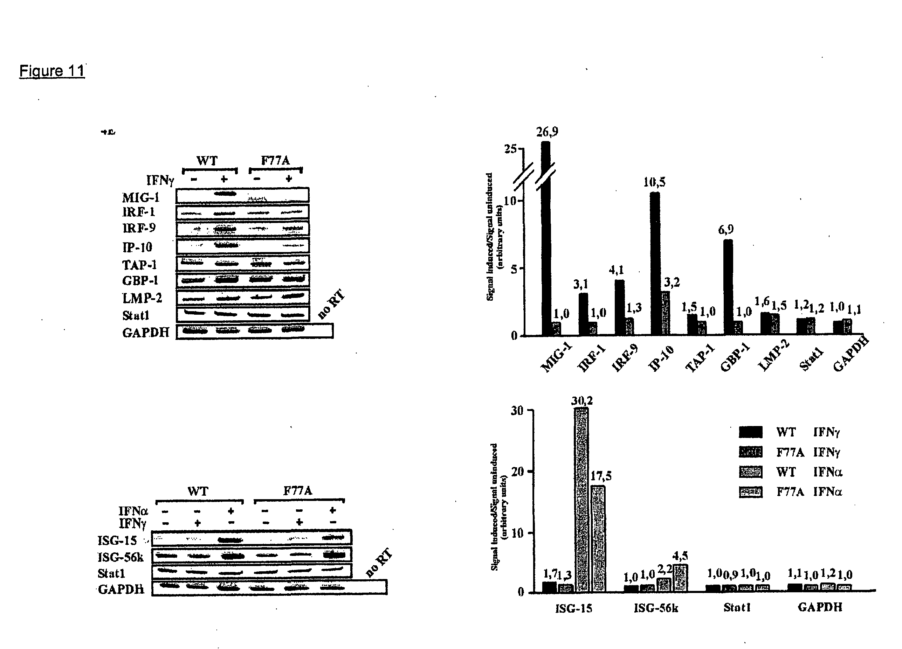

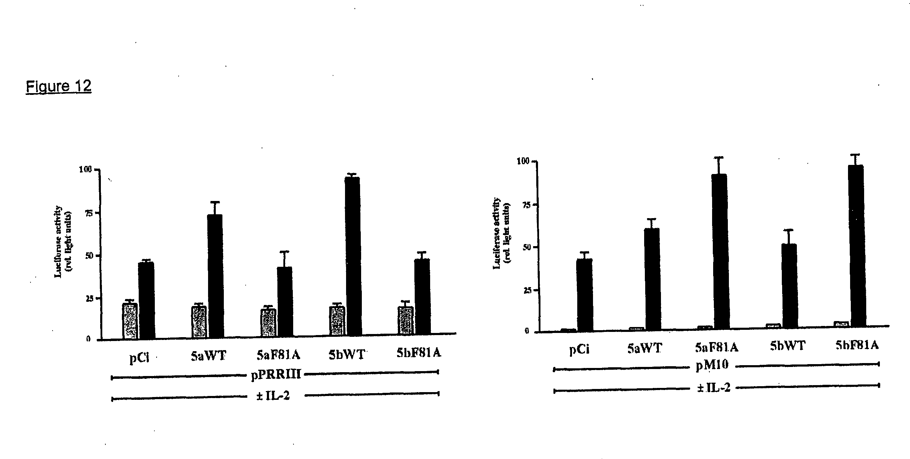

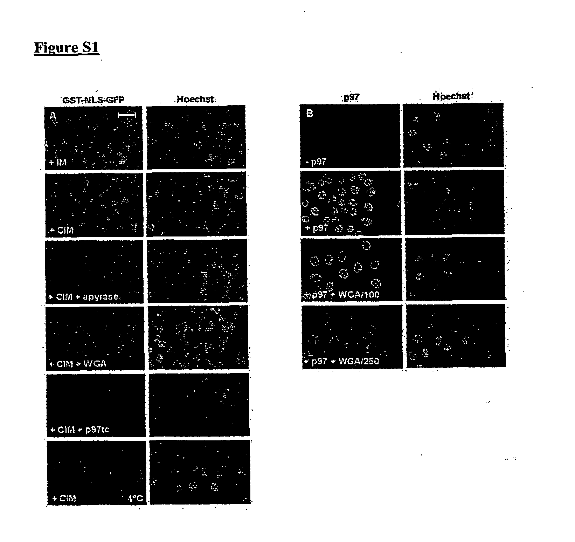

Of the seven genes tested, only LMP-2 expression was unaffected by the N-domain mutation of Stat1, whereas the others showed a strongly diminished IFNγ-responsiveness in cells expressing Stat1F77A. Strong effects were to be seen with the MIG-1, IRF-1, IRF-9, and GBP-1 genes, which had lost their IFNγ-responsiveness in cells expressing the Stat1 mutant protein.
The importance of tetramer formation for gene induction in a natural setting was further evaluated by scoring gene induction in response to IFNα. Contrary to IFNγ, which signals via a Stat1 homodimer, stimulation of cells with IFNα induced the additional formation of a ternary complex called ISGF-3, consisting of a Stat1/Stat2 heterodimer and IRF9, which binds to an unrelated recognition site termed interferon-stimulated response element (ISRE). Expression of the ISG-15 gene was highly induced by IFNα, and mutation of F77 reduced its IFNα response by one half, whereas the weak induction of ISG-56 was not adversely affected by the N-domain mutation (Figure 11).
Due to the association of added library substances with Stat mutants, changes in the transcription yield of target genes may occur and can be easily detected.

### 2. Example

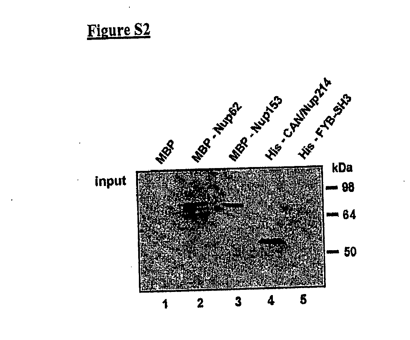

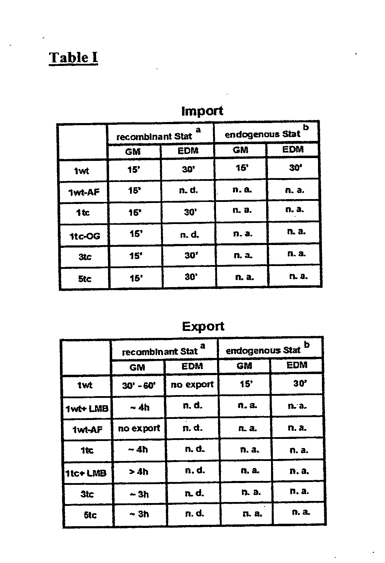

The plasmids pCIStat5a and pCIStat5b were described (John *et al.,* Mol. Cell. Biol. 19, 1910-1918 (1999)). Site specific mutagenesis of these plasmids according to manufactures' instructions (QuikChange™, Stratagene; MORPH site-specific plasmid mutagenesis kit, 5'-3' Inc.) generated the respective N-domain mutants Stat5aF81A and Stat5bF81A.
For reporter gene assay, 4*10⁶ 293T cells were co-transfected with the following DNAs: 2 µg IL-2 receptor β, 0.5 µg γ_{c}, 0.25 µg Jak3, 10 ng of each of the wild type Stat5 (control), Stat5aF81A or Stat5bF81A mutant expression plasmids and 1 µg of PRRIII-E1b or M10-E1b-luciferase reporter construct as described (John *et al.,* Mol. Cell. Biol. 19, 1910-1918 (1999)). Either the weak natural promoter PRRIII (Soldani *et al.,* Mol. Cell. Biol. 20, 389-401 (2000)) for the possible screening of transcription activators or the strong synthetic promoter, the PRRIII derivative M10 (Vinkemeier *et al*., EMBO J. 15, 5616-5626 (1996)), for the possible screening of transcription inhibitors was applied to drive the luciferase gene. After 48 hours cells were spread on a microtiter plate containing a library of substances to be screened. The Stat5 transport to the nucleus was induced by administering 2 nM human interleukin 2 (IL-2) overnight firstly leading to nuclear accumulation and secondly to reporter gene expression (Figure 12).
Due to the association of added library substances with Stat mutants, changes in the expression level of the luciferase gene may occur and are monitored via luminescence according to manufacturer's protocols (Promega).

### 3. Example

In this control study we examined the behavior of various unphosphorylated Stat proteins in the absence of cytokine stimulation. Unphosphorylated Stats, preferably Stat1, can enter the nucleus independently of p97 by an unknown mechanism. As generally only molecules of less than 30 kD can freely cross the nuclear pore complex (NPC) by passive diffusion, the signal-independent nuclear import of unphosphorylated Stats (Mr > 87 kD) requires specific interactions with either the NPC or another intermediary carrier. Here, it was demonstrated that Stats can undergo rapid translocation through the nuclear pore in a cytosol-unassisted and carrier-independent manner that does not require metabolic energy. In the meaning of the invention, Stats derived form any Stat wild type or fragments thereof shuttle between cytosol and nucleoplasm, preferably Stat1, Stat3 and Stat5, more preferably Stat1 and most preferably Stat1tc (representing a truncated Stat1 protein lacking the NH₂- and the COOH-terminal domain). The direct binding of Stats to nucleoporins (Nups) indicated that unphosphorylated Stats migrate into the nucleus via specific molecular interactions with components of the NPC. In the meaning of the invention, these components of the NPC comprise nucleoporins and fragments thereof, preferably Nup153 and Nup214. The nucleoporins Nup153 and Nup214 (the number refers to the molecular weight) were preferred chosen because of the exposed arrangement within the nuclear pore making heterologous intermolecular interactions possible, their detailed characterization and high expression yields for subsequent *in-vitro* investigations. In another embodiment of the present invention, molecules which are structurally and functionally homologous to nucleoporins interact with Stats.
The invention also relates to a Stat mutant which could preclude the aforementioned nuclear import. In the meaning of the present invention, a Stat molecule derived form any Stat wild type or fragments thereof is mutated, preferably Stat1, Stat3 and Stat5, more preferably Stat1 and most preferably Stat1tc. Furthermore, at least one amino acid is mutated within the linker domain of the Stat molecule, preferably cysteines, more preferably cysteine in position 543 of Stat1 or in the equivalent position of homologous proteins. Possible mutations comprise the deletion, insertion, substitution and/or modification of at least one amino acid, preferably a single residue. Possible modifications are alkylation, arylation and acetylation, preferably the alkylation. In a specific embodiment of this invention, the amino acid Cys543 in Stat1 or in the equivalent position of homologous proteins is alkylated. The phenotype of shuttle loss can be effectively used as negative control in bioassays requiring shuttling. Preferably, the phenotype represents a test within gene induction assays, like the two-hybrid system.

Cells were grown in complete growth medium. LMB (5 ng/ml, Sigma) was added to the cells 60 min before microinjection. For energy depletion studies, HeLa-S3 or NIH-3T3 cells were washed with PBS, and incubated in serum- and glucose-free DMEM (GIBCO). This medium was supplemented with 10 mM sodium azide and 10 mM 2-deoxy-*D*-glucose (energy depletion-medium, EDM) and left on the washed cells for 2 h. Subsequently, the cells were either fixed in methanol for 6 min at -20° C, or microinjected with antibodies or recombinant protein and the incubation in EDM was continued for the indicated times. Alternatively, after 2 h in EDM the cells were kept in complete growth medium for 5 h before fixation. Stat1 was detected by immunocytochemistry with antibody E-23 (0.2 µg/ml; Santa Cruz).

For expression in bacteria, the cDNAs encoding truncated human Stat1 (aa 130-712), mouse Stat3 (aa 136-716), or sheep Stat5 (aa 138-704) were amplified by PCR and cloned into the EcoRI and BamHI sites of pASKIBA3 (IBA, Göttingen). The cDNA encoding aa 45-462 of p97 was PCR-amplified and cloned into pGEX-5X-2 (Pharmacia). MBP fusion proteins of the FG repeat regions of human Nup62 (aa 1-308, a kind gift of E. Hurt) and human Nup153 (aa 333-618, a kind gift of B. K. Felber) were prepared after PCR-amplification of the respective cDNAs and cloned into the BamHI site of pMal-2CX (NEB). PCR reactions were performed with Vent-proofreading polymerase (NEB). Site-directed mutagenesis was performed with the Quick-change kit (Stratagene). Baculovirus transfer vectors (pFastBac) encoding human Stat1wt (aa 1-746) or the Tyr701Phe mutant and vectors for bacterial expression of GST-NLS-GFP and GST-NES-GFP have been described. Expression constructs for His-tagged human p97 (aa 1-878) and human Nup214 (aa 1549-2090) were kind gifts of U. Kutay and M. Fomerod, respectively.

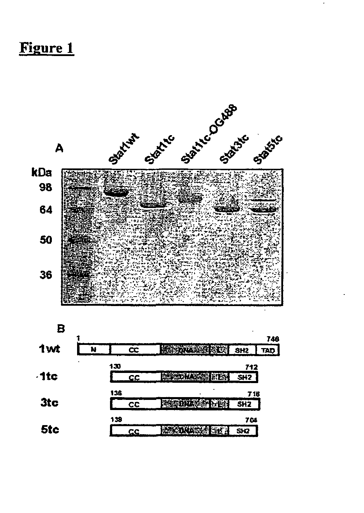

Expression, purification, alkylation, and fluorescent labeling of recombinant proteins were performed as follows. Expression of Stat1wt and Stat1Y701F in baculovirus-infected Sf9 insect cells, the purification and tyrosine phosphorylation were done as previously described. Protein expression in BL21p(Lys)S bacteria was induced at an OD₆₀₀ of 0.8 with 1 mM anhydrotetracycline (Acros, pASKIBA3 vector), or IPTG (1 mM for pGEX and 0.3 mM for pMal vectors, respectively). Protein expression was at 18° C for 15 h (Strep-tagged proteins), or at 30° C for 5 h or 3 h with GST- or MBP-tagged proteins, respectively. Cells were lysed by sonication in PBS containing 1% Triton X-100, 2 mM EDTA, 2 mM EGTA, 5 mM DTT. Proteins were single-step purified by virtue of their Strep- or GST-tag, respectively, as recommended by the manufacturers (IBA; Pharmacia). Where indicated, purified proteins were phosphorylated alkylated with NEM (20 mM) as described. Proteins were concentrated by ultrafiltration (Centriprep, Millipore) in injection buffer containing 5 mM DTT; protein concentrations were determined by UV spectroscopy (extinction coefficient 1.25 for Stat1wt, 1.27 for Stat1tc, 1.24 for Stat3tc, and 1.43 for Stat5tc), or by Coomassie dye-binding (Biorad) with a BSA standard. Proteins were quick-frozen on dry ice and stored at -80° C. Fluorescence labeling of Stat1wt and Stat1tc was done with succinimidyl esters of Alexa Fluor 594 or Oregon Green 488 as described by the manufacturer (Molecular Probes) at a molar protein:dye ratio of 1:5. Efficiency of labeling was close to 100% as judged by mobility shift in SDS-PAGE (Figure 1). His-tagged p97 was expressed and purified as described.

Microinjections of antibodies (at 0.15 mg/ml in PBS) and concentrated recombinant proteins (at 0.5-1 mg/ml) were as described. Injection site markers were used at a concentration of 0.5-1 mg/ml, WGA (Sigma) was co-microinjected at a concentration of 0.5 mg/ml. Immunocytochemistry was with monoclonal Stat1 antibody C-136 (0.2 µg/ml; Santa Cruz) or anti-Strep-tag II rabbit polyclonal antibody (0.2 µg/ml; IBA).

Import assays with permeabilized cells were perfomed as follows: Adherent HeLa-S3 cells were grown on coverslips and permeabilized with 40 µg/ml digitonin (Roche) in transport buffer (TB, 20 mM Hepes, pH 7.3, 110 mM KOAC, 2 mM Mg(OAC)₂, 1 mM EGTA, 2 mM DTT, Complete protease inhibitors (Roche)) for 6 min on ice. Subsequently, the cells were washed in TB twice. Incubation with 20 µl import mix was performed at RT for 60 min or as indicated. The cytosol-free import mix (IM) contained TB supplemented with 10 mg/ml BSA and one or several of the following purified proteins as indicated: 1 µM Stat1tc, 1 µM Stat3tc, 1 µM Stat5tc, 1 µM Oregon Green 488- or Alexa Fluor 594-labeled Stat1tc, 15 µM Stat1wt, 0.8 µM GST-NLS-GFP, or 0.8 µM full-length His-tagged p97. The complete import mix (CIM) contained 75% (v/v) rabbit reticulocyte lysate (Promega), 25% IM, and an energy regenerating system (0,5 mM ATP, 0,5 mM GTP, 10 mM creatine phosphate, 30 U/ml creatine phosphokinase). For WGA treatment, digitonin-permeabilized cells were incubated for 10 min on ice in IM containing 250 µg/ml WGA or as indicated, before washing and addition of the import mix. For ATP depletion, IM or CIM were preincubated for 15 min on ice in the presence of 0.8 U/ml apyrase (Sigma) before addition to the permeabilized cells. For competition assays, 16 µM truncated p97 (aa 45-462) or 20 µM full length Stat1 was added to IM and left on the cells for 45 min. After the import reaction the cells were washed with ice-cold TB and fixed for 10 min at RT with 3.7% para-formaldehyde in PBS, before permeabilization with 0.2% Triton X-100 in PBS (2 min at RT). Truncated Stats were detected with Strep-tag II antibody (0.17 µg/ml); for Stat1wt antibody C-136 was used (0.07 µg/ml); and His-tagged p97 was detected with anti-His antibody (0.25 µg/ml; Abcam). Fixed cells were mounted in TB containing 50% glycerol, 1% (w/v) NaN₃, 1 % (w/v) Diazobicyclo (2,2,2)-odone.

*In vitro* binding assay was perfomed as follows: Bacterial lysates (containing approx. 6 µg NPC proteins or MBP) and 6 µg purified His-tagged control protein (tandem SH3 domains from human FYB; a kind gift of C. Freund) were resolved by 10% SDS-PAGE and transferred to a nitrocellulose membrane. The membrane was rinsed in transport buffer (20 mM HEPES, pH 7.4, 110 mM KOAc, 2 mM Mg(OAc)₂, 0.5 mM EGTA, 2 mM DTT, 0.5% Tween-20, Complete protease inhibitors), and then blocked in this buffer plus 5% (w/v) non-fat milk for 1 h at 4°C. Recombinant full length Stat1 was added at 3 µg/ml, and the incubation continued for 16 h at 4°C. Subsequently, the blot was rinsed three times with transport buffer and subjected to Western analysis using anti-Stat1 antibody C-136 (0.2 µg/ml).

Fluorescence microscopy and fluorescence quantification were performed as follows: Conventional and confocal fluorescence analysis and quantification of immunofluorescence intensities were performed as described. The data were analysed by ANOVA followed by Tukey multiple comparison tests. Differences were considered statistically significant at p < 0.05.

Various recombinant Stat proteins were prepared to gain insight into the cytokine-independent nucleocytoplasmic translocation of these transcription factors (Figure 1). Full length Stat1 was isolated from baculovirus infected insect cells. A stable and well-characterized truncated variant, Stat1tc, which lacks both the N-domain of 129 residues and the C-terminal transactivation domain of 38 residues was expressed in bacteria, as were analogous mutants of Stat3 and Stat5 (Mr > 65 kD). The truncated Stat proteins were purified by virtue of a small C-terminal Strep-tag, which was also useful for indirect immunocytochemical detection. The recombinant proteins were microinjected into unstimulated cell lines or used for import assays with permeabilized cells. In addition, endogenous Stat1 was targeted by antibody microinjection to reveal the flux rates of the native protein.

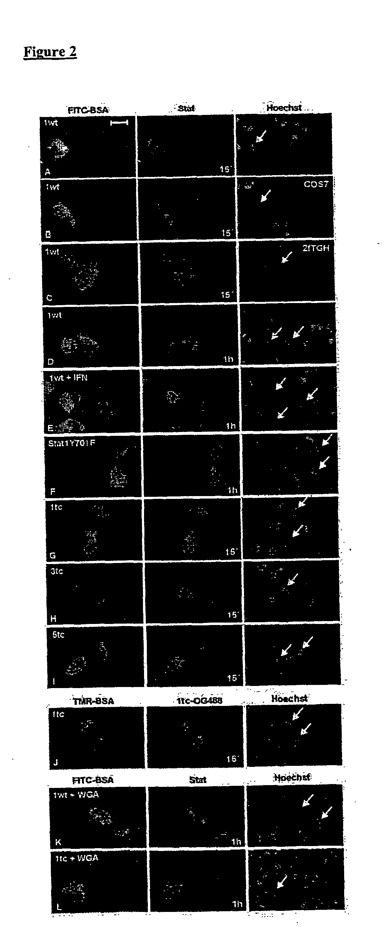

Microinjected Stat1, Stat3, and Stat5 rapidly migrate into the nucleus of unstimulated living cells. Several unstimulated cell lines were microinjected into the cytosol with full length unphosphorylated Stat1. In HeLa-S3 cells (Figure 2A), COS7 cells (Figure 2B), and 2fTGH cells (Figure 2C) nuclear import occurred with identical velocity, as it took only 15 min to reach pancellular distribution. Thereafter, no further nuclear accumulation could be observed and the nucleocytoplasmic distribution remained stable (Figure 2D). We then compared wild type Stat1 with a tyrosine phosphorylation-defective mutant (Tyr701Phe) and with truncated Stat1tc. As is shown in Figure 2E and F, nuclear translocation of the two Stat1 variant proteins was indiscriminable from wild type and also resulted in a pancellular distribution at equilibrium, which indicated that tyrosine phosphorylation as well as N- and C-termini were dispensable for cytokine-independent nuclear import. Next we tested the import abilities of truncated Stat3 and Stat5, and found that both proteins enter the nucleus with kinetics similar to Stat1 (Figure 2G and H). Notably, the injected Stat3 repeatedly showed a higher steady state concentration in the nucleus than the two other Stat proteins. Additionally, Stat1 and Stat1tc were labeled on lysine residues with fluorescent dyes (either Oregon Green 488 or Alexa Fluor 594) to directly observe their nuclear translocation. As is shown in Figure 21 for Oregon Green 488-labeled Stat1tc, conjugation with fluorescent dyes did not interfere with nuclear import (identical result were obtained with Alexa Fluor 594 and for full length Stat1). To demonstrate exclusive passage of the microinjected material through the nuclear pore, we co-injected wheat germ agglutinin (WGA), a lectin known to inhibit nuclear pore-mediated translocation of macromolecules. Expectedly, passage across the nuclear envelope of both full length and truncated Stat1 was effectively blocked (Figure 2J, K).

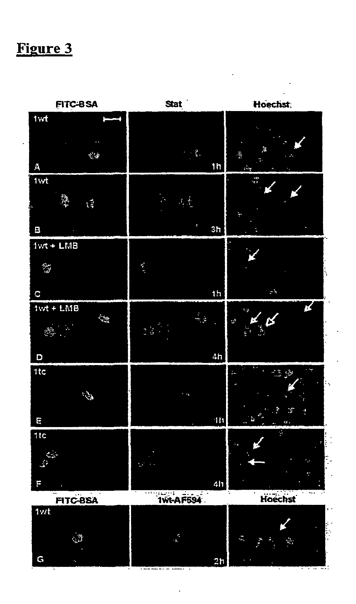

The requirements for the constitutive nuclear import and export of recombinant Stat1 are biochemically distinguishable. Next, recombinant Stat1wt was injected into the nucleus to investigate export kinetics. The microinjected protein quickly left the nucleus and spread evenly across the cell. However, nuclear export appeared to occur slower than nuclear import. It usually took about 30 to 60 min to reach a pancellular distribution (Figure 3A), which then remained stable (observation for another 2 h) (Figure 3B). Nuclear export of Stat1 after cytokine-induced nuclear accumulation is achieved in part via the transport receptor CRM1. It is unclear, however, whether CRM1-dependent nuclear export also functions in the absence of cytokine stimulation. Therefore, cells were treated with the CRM1 inhibitor leptomycin B (LMB) starting 60 min before nuclear microinjection. This treatment attenuated nuclear export, since the recombinant protein was still predominantly nuclear after 1 h (Figure 3C), and a pancellular distribution was achieved not even after 4 h (Figure 3D, filled arrow). Notably, preincubation with LMB followed by cytoplasmic microinjection of Stat1 did not cause its nuclear accumulation (Figure 3D, open arrow). These results show that LMB reduces the constitutive nuclear export of Stat1 in unstimulated cells. We have shown in Figure 2F that removal of N- and C-domains did not influence the nuclear import of Stat1. Contrary, nuclear export of the truncated mutant Stat1tc was strongly diminished, since it took about four hours to achieve an even nucleocytoplasmic distribution (Figure 3E, F), and treatment with LMB reduced the export rate even further (Table I). Similar results were obtained also for truncated Stat3 and Stat5 (Table I). Moreover, fluorescently labeled full length Stat1 was not exported from the nucleus even after 2 h (Figure 3G).

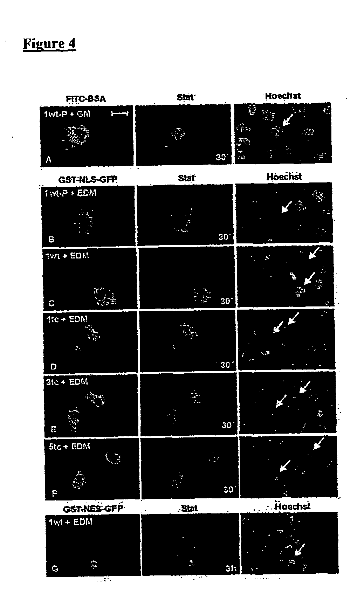

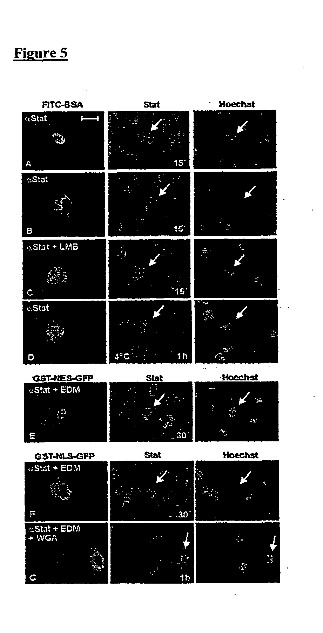

The constitutive nuclear import of recombinant Stat1 continues in energy-depleted cells. Another set of microinjection experiments was performed in living cells that were depleted of ATP by the addition of sodium azide and 2-deoxyglucose. Such treatment was shown to reversibly inhibit classical Ran-dependent nuclear transport by limiting the pool of GTP-bound Ran. In order to demonstrate the energy-depleted status of the microinjected cells, we used different injection markers. Here, fusion proteins of glutathione S-transferase (GST) and green fluorescent protein (GFP) that included at their domain junction either a triple SV40 NLS or a Stat1-derived NES (termed GST-NLS-GFP or GST-NES-GFP, respectively) were co-microinjected with recombinant Stat1. The effective nucleocytoplasmic translocation of the reporter constructs was confirmed in cells growing in full-medium, whereas pre-exposure of cells to azide and 2-deoxyglucose for 2 h prevented the carrier-dependent nuclear translocation of the reporter protein (Figures 4 and 5E, F). Expectedly, the nuclear import of microinjected tyrosine-phosphorylated Stat1wt and Stat1tc was also prevented in energy-depleted cells (shown for Stat1tc in Figure 4A). However, nuclear import of unphosphorylated Stat1tc (Figure 4B) and wild type (Figure 4C) continued under this condition, albeit at a reduced pace, as it now took about 30 min to reach a pancellular distribution after injection into the cytoplasm. Similarly, nuclear import of recombinant Stat3tc and Stat5tc also continued in energy-depleted cells (Figure 4D, E). Notably, the predominantly nuclear localization of Stat3tc seen in cells growing in normal medium was not maintained in ATP-depleted cells (compare Figures 4D and 2G).
Next, the nuclear export of recombinant wild type Stat1 and Stat1tc in energy-depleted cells was examined, and it was found that energy depletion blocked the export from the nucleus of both proteins during a 3 h observation period (shown in Figure 4F for Stat1wt; compare with Figure 3A, B).

Endogenous Stat1 is constitutively shuttling across the nuclear envelope independently of metabolic energy. A different experimental approach was used to investigate the nucleocytoplasmic flux rates also of endogenous Stat1. To this end we performed microinjections of Stat1 antibodies, which has been shown to immobilize the shuttling target antigen and hence cause its accumulation in the microinjected compartment. The results are assembled in Figure 5 and Table I. As is shown in panel A of Figure 5, maximal nuclear accumulation of unphosphorylated Stat1 was seen already 15 min after antibody microinjection into the nucleus. Next, cytoplasmic antibody microinjections were used to estimate the nuclear export rate of the endogenous Stat1 protein. Depletion of nuclear Stat1 was observable as early as 5 min post injection, and maximal depletion was achieved with this technique already after 15 min (Figure 5B). This assay was also used to determine the inhibitory influence of LMB on the nuclear export of endogenous Stat1. However, the antibody microinjection assay did not allow us to record differences, as the nuclear export rate was not measurably diminished in the presence of LMB (Figure 5C). Incubation of the cells at 4°C, however, precluded nuclear export of endogenous Stat1 (Figure 5D).
Nuclear import of the endogenous Stat1 continued also during ATP-depletion, as revealed after nuclear injection of a Stat1 antibody. Already 15 min past antibody microinjection nuclear accumulation was observable, which was maximal after 30 min (Figure 5E). We then explored the nuclear export of endogenous Stat1 under these conditions. This was achieved by cytoplasmic co-microinjection of a Stat1 antibody and the GST-NLS-GFP reporter (Figure 5F). Clearly, nuclear export of endogenous Stat1 continued in azide/2-deoxyglucose-treated cells, as the nuclear compartment was depleted of Stat1 immunoreactivity after cytoplasmic antibody injection. Energy depletion reduced the transport rate by about 50%, as the time required to clear the nucleus of Stat1 had doubled to 30 min. Together, these results indicated that nucleocytoplasmic shuttling of Stat1 continued in the absence of a physiological Ran-GTP pool.

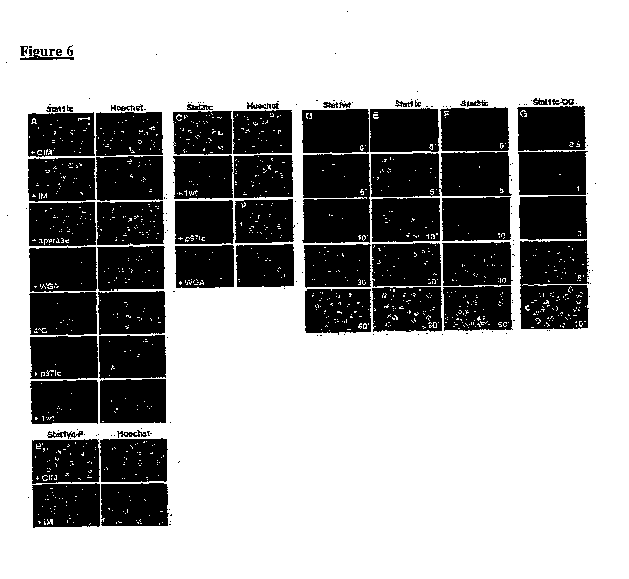

Exclusively unphosphorylated Stats enter the nucleus of digitonin-permeabilized cells in the absence of cytosol. The behavior of Stat proteins was investigated further with a permeabilized cell transport assay. HeLa cells were incubated with a concentration of digitonin (40 µg/ml) that selectively permeabilizes the plasma membrane, thereby releasing cytoplasmic proteins. An extensive validation of this assay was performed with the carrier-dependent import substrate GFP-NLS-GST. As expected, nuclear import required the addition of cytosol and metabolic energy (see Supplemental Figure 1). A similar analysis was also performed for unphosphorylated Stat1. As is shown in Figure 6A, Stat1tc entered the nucleus in the presence of cytosol and metabolic energy (+CIM, complete import mix). However, nuclear import was even enhanced in the absence of added exogenous cytosol (Figure 6A, +IM, import mix). Pre-treatment of digitonin-permeabilized cells with apyrase to further reduce residual ATP in the absence of added cytosol was without effect on the import of unphosphorylated Stat1tc (Figure 6A, +apyrase), in contrast to tyrosine-phosphorylated Stat1tc, which was incapable to enter the nucleus in the absence of added cytosol (Figure 6B).
Several conditions were found to prevent nuclear import of unphosphorylated Stat1tc in the absence of added cytosol. First, the addition of WGA strongly reduced Stat1 nuclear uptake (Figure 6A; +WGA), as did incubation of the permeabilized cells at 4° C (Figure 6A; 4° C). To further characterize cytosol-independent nuclear translocation, we examined the saturability of the import process. For this experiment, an N- and C-terminally truncated fragment of the import receptor p97 was added to the cytosol-free import reaction. This mutant binds irreversibly to proteins of the nuclear pore and thus obstructs multiple import pathways in a dominant fashion. As is shown in Figure 6A (+p97tc), the inclusion of a 16-fold molar excess of the inhibitory p97 fragment (residues 45-462) completely abrogated nuclear import of Stat1tc. The same result was obtained after adding a 20-fold molar excess of wild type Stat1 to the import reaction (Figure 6A; +1wt).
Cytosol-free import assays with digitonin-permeabilized HeLa cells were succesfully performed also with Stat3tc (Figure 6C). Since both Stat proteins could enter the nucleus by a carrier-free mechanism, we tested whether they also compete for the same NPC binding sites. This was done by adding a 20-fold molar excess of wild type Stat1 to the Stat3tc import reaction, which resulted in a strong suppression of Stat3tc nuclear import (Figure 6C; +1wt). Similarly, addition of a 16-fold molar excess of truncated p97 also reduced nuclear import of Stat3tc, as did pre-incubation with WGA (Figure 6C; labeled +p97tc and +WGA, respectively).
To estimate the nuclear entry rates of Stat1wt and truncated Stat1 and Stat3 in digitonin-permeabilized cells, a time-course experiment was performed. As is shown in Figure 6D-F, all three proteins entered the nucleus with similar kinetics. Already after 5 min the Stat proteins were detectable at the nuclear rim and inside some nuclei, and after 10 min the Stats had accumulated in most of the nuclei. The accumulation phase continued and reached a plateau between 30 and 60 min. Interestingly, we noted that the import rate of Stat1 and Stat3 differed among individual nuclei (Figure 6D-F). Incorrect cell permeabilization leading to rupturing of the nuclear membrane is an unlikely explanation for this, since staining of digitonin-permeabilized cells with labeled concanavalin-A was limited to the cell membrane. Moreover, the cytosol-free nuclear import of p97 did not differ among individual nuclei of permeabilized cells (see Supplemental Figure 1 B). Of note, fluorescent labeling increased both the transport rate and the homogeneity of Stat1 nuclear import (shown for Stat1tc in Figure 6G). We also attempted to establish cytosol-free nuclear import for Stat5tc. While about 10% of the nuclei showed nuclear import of Stat5tc, the remaining cells displayed strong labeling in the extranuclear space of what appeared to constitute precipitated Stat5tc protein.
These results indicate that Stat1, Stat3, and Stat5 have the ability to migrate into the nucleus by themselves in the absence of metabolic energy or added transport factors. They share binding partners in the NPC among each other and with the import receptor p97.

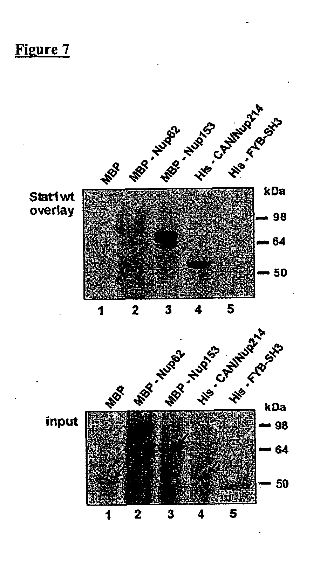

Stat1 binds to nucleoporin Nup153 and Nup214, but not Nup62. The above data are not compatible with a carrier-mediated nuclear translocation process. Rather, they are characteristic for a carrier-free mechanism that occurs through direct interactions between the Stats and constituents of the nuclear pore. A distinctive feature of numerous NPC proteins is the presence of Phenylalanine/Glycine (FG)-rich repeat motifs, which provide interaction sites for transport factors. Since Stats and p97 appeared to share binding sites on nuclear pore proteins, we examined whether Stat1 could also interact with constituents of the nuclear pore. FG repeat-containing parts of His-tagged Nup214 (residues 1549-2090), Nup62 (residues 1-308), and Nup153 (residues 333-618; the latter two were fusion proteins with maltose-binding-protein, MBP) were expressed in bacteria and detected by immunoblotting with the monoclonal antibody 414 (Supplemental Figure 2). The bacterial lysates were resolved by denaturing SDS-PAGE (Figure 7, lower panel) and subsequently blotted on nitrocellulose. After extensive incubation in renaturing buffer, the immobilized proteins were incubated with wild type Stat1, the binding of which was detected by Western blotting (Figure 7, upper panel). No binding of Stat1 was seen with MBP and a His-tagged control protein, but Stat1 bound strongly to both Nup214 and Nup153. Contrary, no binding was detected to the FG repeat region of Nup62. These results suggested that Stat1 is able to bind directly to the FG repeat region of Nup153 and Nup214, supporting a model in which unphosphorylated Stat1 engages in carrier-free nucleocytoplasmic shuttling through direct interactions with components of the nuclear pore.

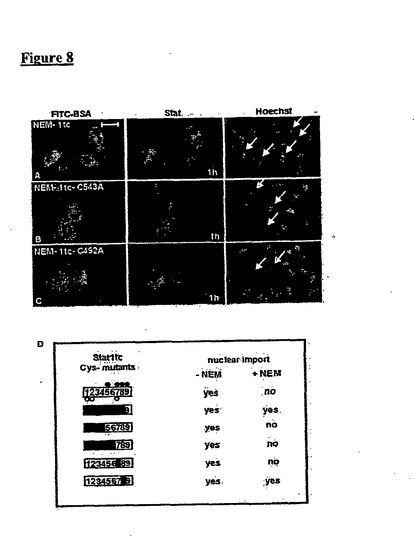

The linker domain of Stat1 plays a role in cytokine-independent nuclear translocation. Stat1 contains highly reactive cysteine residues which can cause aggregation of recombinant purified protein. This problem can be overcome by blocking reactive SH-groups with N-ethylmaleimide (NEM), which has been shown to leave *in vitro* DNA binding undisturbed, and this treatment also did not influence nuclear import of full length Stat1. However, when truncated Stat1 was alkylated and microinjected into cells, we could no longer detect nucleocytoplasmic shuttling, as both import and export were strongly diminished (Figure 8A). Mutation of eight out of the nine cysteine residues of human Stat1tc prevented NEM from inhibiting nuclear import (see summary in Figure 8D), indicating that cysteine alkylation caused the inhibitory effect. Further experiments unambigously demonstrated that mutation of Cys543, which is located in the linker domain, was responsible for rescuing Stat1 from the shuttling defects caused by NEM (Figure 8B). Mutation of other cysteine residues did not prevent NEM from inhibiting nuclear translocation of Stat1 (shown for Cys492 in Figure 8C). Mass spectrometry confirmed alkylation of recombinant Stat1tc in position 8 (Cys543), and also of positions 5 (Cys324) and 9 (Cys577) (see Figure 8D). Interestingly, mutation to alanine or arginine of residue 543 was without adverse effects on nuclear import.

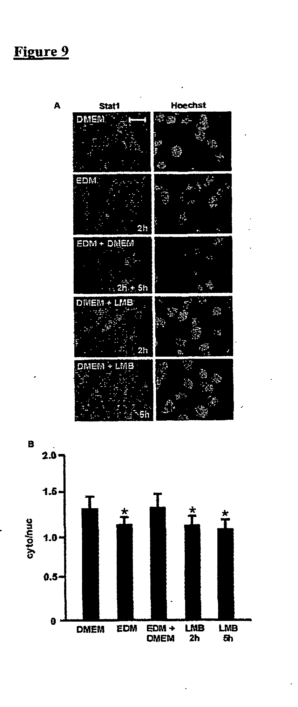

Carrier-dependent and -independent pathways cooperatively determine the subcellular distribution of Stat1 in resting cells. It is currently unknown how the Stats achieve their predominantly cytoplasmic localization in unstimulated cells. As is shown above, before stimulation with cytokines Stat1 enters the nucleus independent of metabolic energy and transport factors. Export of Stat1 from the nucleus, however, can occur via two pathways, only one of which functions without metabolic energy, whereas the second pathway requires an NES, the exportin CRM1 and metabolic energy. Therefore we used energy-depletion and employed the specific CRM1 inhibitor LMB to suppress the NES-dependent active nuclear export of Stat1. As is shown in Figures 9A, exposure of 3T3 cells to energy depletion medium for 2 h reduced the cytoplasmic accumulation of Stat1, and a statistically significant relocation to the nucleus resulting in a pancellular distribution was observed (Figure 9B). Expectedly, the nuclear relocation of Stat1 was reversible, since the subsequent incubation in full growth medium for 5 h restored the cytoplasmic accumulation of Stat1 (Figure 9A). Treatment of cells with the CRM1 inhibitor LMB also caused relocalization and pancellular distribution of Stat1. Importantly, prolonged exposure to LMB for 5 h had the same effects and did not induce nuclear accumulation (Figure 9A, B).

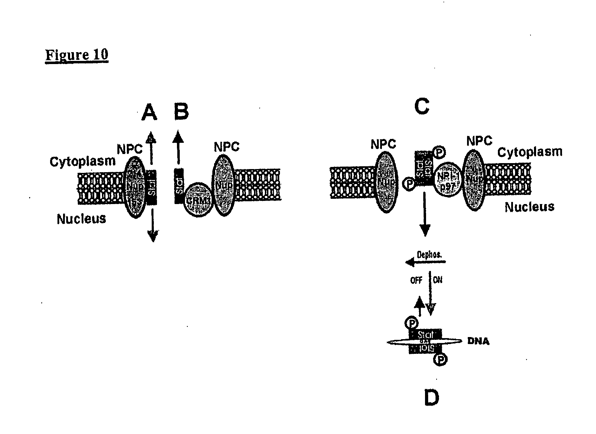

In the present study we have investigated both *in vitro* and *in vivo* the nucleocytoplasmic translocation of Stats before their activation. The results shown here reveal that unphosphorylated Stat1, Stat3 and Stat5 are shuttling proteins that rapidly traverse the nuclear envelope. Several lines of evidence indicated that this is a process distinct from the conventional import mechanism described for tyrosine-phosphorylated Stat1 (Figure 10). 1) All transport processes known to date that depend upon transport factors rely on metabolic energy *in vivo.* Nuclear translocation of unphosphorylated Stat1, Stat3, and Stat5, however, continued in energy-depleted cells, whereas the translocation of tyrosine phosphorylated Stat1 or of a transport factor-dependent reporter construct did not (Figure 4, 5E, F). 2) Nuclear import of unphosphorylated Stat1 and Stat3 was readily observable in digitonin-permeabilized cells in the absence of added cytosol (Figure 6A, C). Contrary, tyrosine-phosphorylated Stat1 or a p97-dependent reporter construct could not enter the nucleus in this system (Figure 6B and Supplemental Figure 1A). 3) Unphosphorylated Stat1 was demonstrated to directly interact with the FG repeat region of Nup153 and Nup214, but not Nup62 (Figure 7). These results are compatible with a model describing nucleocytoplasmic translocation of unphosphorylated Stats as a carrier-independent process that relies on direct interactions between Stat proteins and the nuclear pore (Figure 10). The carrier-free nuclear import of the Stats is rapid and saturable. Nuclear import in digitonin-permeabilized cells or after cytoplasmic microinjection was detectable already after 5 min, and trapping of endogenous Stat1 by antibody microinjection in the nucleus of resting HeLa cells resulted in nuclear accumulation after about 15 min. Contrary to the results presented here for wild type Stat1, examination of GFP-tagged Stat1 with FLIP (Fluorescence Loss in Photo bleaching) indicates that the nuclear and cytoplasmic pools do not exchange rapidly. However, in unstimulated cells GFP-tagged Stat1 traverses the nuclear membrane much less efficiently in comparison to the wild type (manuscript in preparation).
Active nuclear export has been demonstrated to participate in the termination of the interferon-induced nuclear accumulation of Stat1. Here, we extend these data and show that both active, CRM1-dependent and carrier-free nuclear export occur simultaneously regardless of cytokine stimulation, as indicated by the protracted export of nuclear-microinjected Stat1 during LMB-treatment (Figure 3C, D). Several observations suggested that nuclear import of unphosphorylated Stat1, on the other hand, is a carrier-free process. Namely, the cytoplasmic microinjection of fluorescently labeled Stat1 that cannot leave the nucleus did not result in nuclear accumulation, but import ceased when a pancellular distribution was achieved (Figure 21). Similarly, the specific inactivation of CRM1-mediated export did not cause nuclear accumulation either (Figure 3D). Both results argue against constitutive active nuclear import. In addition, no evidence for a functional NLS was found in unphosphorylated Stat1. We therefore propose a model in which diffusion-controlled import and energy-consuming export contribute to the observed accumulation of Stat1 in the cytoplasm of unstimulated cells. In line with this model we found that the cytoplasmic accumulation of Stat1 was significantly diminished upon ATP-depletion or exposure of resting cells to the CRM1 inhibitor LMB (Figure 9). Similar observations were previously made also for Stat2, Stat3 and Stat5. We thus conclude that the influx rate of Stat1 into the nuclei of unstimulated cells is influenced by its concentration gradient between cytosol and nucleoplasm.
Given the active CRM1-dependent nuclear export, the steady-state distribution of microinjected Stat1 should be predominantly cytoplasmic. However, we observed a pancellular distribution at equilibrium. This could be explained if CRM1 played only a minor role in comparison to the transport capacity of carrier-free translocation. The following observations support such an assumption. First, the cytoplasmic accumulation of Stat1 is not very pronounced in most cell lines. Moreover, nuclear export of endogenous Stat1 both before (Figure 5C) and during interferon treatment of cells was not measurably decreased by LMB, which certainly reflects also the limitations of our microinjection assay. And third, LMB prolonged nuclear accumulation of Stat1 only modestly. However, the Stat proteins are likely to differ in the extent to which these pathways determine their overall transport rate. Contrary to Stat1, a constitutive carrier-dependent import signal was described for Stat3. Accordingly, the cytoplasmic microinjection of Stat3tc resulted in its nuclear accumulation (Figure 2G), but an even nucleocytoptasmic distribution resulted if the cells were cultured in energy-depleted medium (Figure 4D).

The structural requirements that enable the nucleocytoplasmic shuttling of unphosphorylated Stats are likely to be complex. Similar to observations that were previously made with importin p97, where alkylation with NEM precluded binding to the nuclear pore, also the translocation of Stat1 was sensitive to NEM. Alkylation of a single variant cysteine residue in the linker domain precluded nuclear translocation and thus implicated this rather non-descript region of Stat1 in nuclear transport. Further work is also necessary to explore the role of the Stat N- and C-domains, which were dispensable for nuclear import, but the presence of which accelerated nuclear export. These observations as well as the competition data, which indicated that the Stat proteins share NPC-binding sites among themselves and with p97 (Figure 6A, C) raise the issue of specificity in carrier-free protein translocation. Besides the truncation of Stats several other conditions affected transport in a direction-specific manner. Energy depletion and fluorescent labeling specifically blocked the nuclear export of Stat1. Also, for unknown reasons the import rates of both Stat1 and -3 differed among individual nuclei of digitonin permeabilized cells. Addition of ATP, which remedied a similar problem in carrier-free nuclear translocation of importin-α, or using recombinant protein from insect cells instead of bacteria had no effect on nuclear import. However, labeling of Stat1 with fluorescent dyes, while preventing export (Figure 3G), made nuclear import more homogenous (Figure 6G). Of note, several experiments indicated that the shuttling rate of endogenous Stat1 exceeded that of the microinjected recombinant protein (Table I). Therefore, it is conceivable that posttranslational modifications influence the translocation through the nuclear pore, possibly even in a direction-specific manner. Such mechanisms could also account for cell-type or Stat-specific differences in the subcellular distribution of members of this protein family.
In summary, our results indicate that a dynamic equilibrium of constitutive nuclear export and import maintains the steady-state distribution of Stats both before and after cytokine stimulation of cells. Thus, in light of the constant cycling that characterizes the Stat transcription factors, the cytokine signal transduction is best described as a "continous signaling cycle" rather than a linear pathway that starts at the cell membrane and ends in the nucleus.
Figure 1. Recombinant Stat proteins. (A) SDS-PAGE (7%) analysis of the Stat protein preparations used in this work (1 µg of each). Molecular size markers are indicated to the left. OG488, Oregon Green 488. (B) Schematic representation of the respective Stat proteins. The truncated mutants were expressed with a C-terminal Strep-tag of the sequence SAWSHPQFEK (single letter code).The Stat proteins were of human (Stat1), mouse (Stat3), or sheep (Stat5) origin. N, N domain; CC, coiled coil domain; DNA, DNA binding domain; L, linker domain; SH2, SH2 domain; TAD, transactivation domain.
Figure 2. Unphosphorylated recombinant Stat proteins translocate into the nucleus of unstimulated cells. HeLa-S3 cells (except for panels B and C) were used. The injection site is indicated by the location of co-microinjected FITC-labeled or Tetramethyl-rhodamine (TMR)-labeled BSA. Nuclei were stained with Hoechst dye. Arrows indicate injected cells. (A-C) Cytoplasmic microinjection of purified Stat1wt into Hela cells (A), COS7 cells (B), or 2fTGH cells (C). After 15 min the cells were fixed and Stat1 was detected by immunocytochemistry with a specific antibody. (D) Identical experimental setup as in (A), but microinjected cells were fixed after 60 min (E-H). Cytoplasmic microinjection of full length tyrosine mutant Y701F (E), or truncated Stat1tc (F), Stat3tc (G), or Stat5tc (H). Fixation was 15 min post injection, truncated Stats were detected with Strep-tag antibody. (I) Cytoplasmic microinjection of Oregon Green 488-labeled Stat1tc. Cells were fixed after 15 min followed by direct fluorescence microscopy. (J, K) Cytoplasmic co-microinjection of WGA and Stat1wt (J) or Stat1tc (K). After 1 h the cells were fixed and Stat1 variant proteins were detected by immunocytochemistry as described. Bar, 20 µm.
Figure 3. Unphosphorylated recombinant Stat proteins exit the nucleus of unstimulated cells. The injection sites of the HeLa cells are indicated by co-microinjected FITC-labeled BSA. (A, B) Recombinant Stat1wt was injected into the nucleus. After 1 h (A) or 3 h (B) immunocytochemistry with a Stat1-specific antibody was performed on fixed cells. (C, D) Microinjection of Stat1wt into cells pretreated for 1 h with LMB. After nuclear (filled arrow) or cytoplasmic (open arrow) microinjection the cells were further incubated with LMB for 1 h (C) or 4 h (D) before fixation and immunocytochemistry with a Stat1-specific antibody. (E, F) Stat1tc was injected into the nucleus and the cells were subsequently incubated for 1 h (E) or 4 h (F), before fixation and immunocytochemistry with Strep-tag antibody. (G) Nuclear microinjection of Alexa Fluor 594-labeled Stat1wt, followed by incubation for 2 h, fixation, and direct fluorescence microscopy. Bar, 20 µm.
Figure 4. The nucleocytoplasmic translocation of recombinant Stat proteins continues in energy-depleted cells. HeLa cells were kept in energy depletion medium for 2 h before microinjection (EDM). (A-E) Cytoplasmic microinjection of tyrosine-phosphorylated Stat1tc (A), or unphosphorylated Stat1tc (B), Stat1wt (C), Stat3tc (D), or Stat5tc (E). Injection site and energy depletion are indicated by the location of co-microinjected GST-NLS-GFP. The cells were fixed at the indicated times after injection and Stats were detected by immunocytochemistry (see Figure 2). (F) Nuclear co-microinjection of recombinant Stat1wt and GST-NES-GFP, followed by 3 h of incubation in EDM. Then, cells were processed for immunocytochemistry as before. Bar, 20 µm.
Figure 5. Nucleocytoplasmic translocation of endogenous Stat1 in normal and energy-depleted HeLa cells. Stat1 antibodies were microinjected followed by immunocytochemical detection of Stat1. (A) Microinjection of Stat1 antibodies into the cell nucleus with fixation after 15 min. The injection site is marked by co-microinjected FITC-BSA. (B) Identical with (A), except that the antibodies were microinjected into the cytoplasm. (C) Identical with (B) except that the cells were incubated with 5 ng/ml LMB starting 1 h before microinjection. (D) Identical with (B), except that the cells were kept at 4°C for 60 min both before and after cytoplasmic microinjection. (E) Stat1 antibodies were microinjected into the nucleus of cells after a 2 h preincubation in EDM. Injection site and energy depletion are indicated by co-microinjected GST-NES-GFP. The cells were fixed 30 min after microinjection. (F) Identical with (E), except that the Stat1 antibody was microinjected into the cytoplasm. Injection site and energy depletion are indicated by co-microinjected GST-NLS-GFP. Bar, 20 µm.
Figure 6. The nuclear import of Stat proteins continues in cytosol-depleted digitonin-permeabilized cells. HeLa-S3 cells were treated with 40 µg/ml digitonin, washed, and then incubated with 20 µl of import mix (IM; protein-free transport buffer supplemented with 10 mg/ml BSA) at RT unless noted otherwise. The nuclei are stained with Hoechst dye. Unlabeled Stat proteins were detected immunocytochemically in fixed cells. (A) Permeabilized HeLa cells were incubated for 60 min with 1 µM unphosphorylated Stat1tc in IM. Additionally, the import reaction was performed on ice (4°C), or after preincubation with 250 µg/ml WGA (+WGA). Where indicated, the following additions were made to IM. Rabbit reticulocyte lysate (75%) and an energy regeneration system (+CIM); 0.8 U/ml apyrase (+apyrase); 16 µM truncated p97 (residues 45-462) fused to GST (+p97tc); 20 µM Stat1wt (+1wt). (B) Import reaction with tyrosine-phosphorylated Stat1tc in IM. (C) Identical experimental setup as in (A), but with Stat3tc replacing Stat1tc. (D-G) Time-course of carrier-free nuclear import of Stat1wt (D), Stat1tc (E), Stat3tc (F), or Oregon Green 488-labeled Stat1tc (G). The import mix contained 1µM of the respective Stat proteins in 1M. In (G) Stat1tc was detected by direct fluorescence microscopy. Note the different time scale. Bar, 30 µm.
Figure 7. Stat1 interacts with FG repeat-containing nucleoporins. (Upper panel) Bacterial lysates containing the indicated fusion proteins (lanes 1-4) or purified His-tagged tandem SH3 domains from FYB (lane 5) were resolved by SDS-PAGE, transferred to a nitrocellulose membrane, and probed with wild type unphosphorylated Stat1. The association of Stat1 with the immobilized proteins was detected by immunoblotting using a Stat1 specific antibody. The lower panel shows the respective Coomassie-blue stained SDS-gel before transfer to a nitrocellulose membrane (input).
Figure 8. Alkylation of cysteine 543 in the linker domain precludes nuclear import of Stat1. (A-C) Purified Stat1tc or the indicated mutants were alkylated by NEM *in vitro* and injected in the cytosol of HeLa cells. After 1 h the cells were fixed and the intracellular distribution of the microinjected proteins was determined by immunocytochemistry. Bar, 20 µm. (D) Summary of the microinjection data and mass spectrometric analyses of alkylated Stat1. Purified bacterially expressed Stat1tc was treated without or with NEM and subsequently injected into the cytoplasm of HeLa cells. The ability to enter the nucleus during an 1 h incubation period is stated. The cysteine residues in position 155, 174, 247, 255, 324, 440, 492, 543, 577 of human Stat1 are numbered 1 to 9 in the diagram to the left. Mutated cysteines (Cys to Ala) are blackened. Open circles denote absence of alkylation, filled circles denote alkylated cysteines as determined by mass spectrometry. For position 7 both alkylated and non-alkylated peptides were found. Peptides covering Cys-positions 3, 4, and 6 were not detected
Figure 9. The inhibition of active nuclear export prevents cytoplasmic accumulation of Stat1. (A) 3T3 cells were left untreated (DMEM) or treated with energy depletion medium for 2 h (EDM) followed by growth medium for 5 h (EDM+DMEM). Alternatively, cells were treated with the CRM1 inhibitor leptomycin B (LMB) for 2 h or 5 h as indicated. Shown is the distribution of the endogenous Stat1 as revealed by immunocytochemistry with a specific antibody and conventional microscopy. (B) Corresponding bar diagram with a quantitative analysis of the ratio of the mean cytoplasmic and nuclear immunofluorescence densities. The median slice (x/y image) of a confocal microscopical image was used to quantify the signals in 13 randomly chosen cells. Statistically significant differences between cells incubating in control (DMEM) or experimental medium are indicated by (*). Bar, 20 nm.
Figure 10. A model of Stat1 nucleocytoplasmic shuttling. (A) Unphosphorylated "latent" Stat1 constitutively shuttles between cytosol and nucleoplasm via direct interactions with the nucleoporins Nup153 and Nup214. The surface of the linker domain of Stat1 is likely to provide the contact surface. (B) Additionally, NES-mediated transport of unphosphorylated Stat1 via CRM1 enhances the export rate and achieves cytoplasmic accumulation. (C) After cytokine-induced receptor activation, Stat1 is tyrosine-phosphorylated and dimerizes, which precludes further carrier-free nucleocytoplasmic cycling. However, a dimer-specific NLS in the DNA binding domain is exposed, and nuclear import occurs in complex with NPI-1 and p97. (D) Until its dephosphorylation, which is inhibited by DNA-binding, Stat1 is retained in the nucleus, thus allowing for the signal-induced nuclear accumulation. GAS, Stat1 binding site.
Figure 11. RT-PCR analysis of endogenous INFα- and IFNγ-responsive genes.
Figure 12. IL-2-reconstituted 293T cells are co-transfected with Stat5 wild type or mutant Stat5aF81A or Stat5bF81A and a luciferase reporter gene. Stimulation without (gray) or with IL-2 is overnight (black). Luciferase expression is activated from a natural PRRIII promoter or an M10 element. The diagrams represent the ground expression level which is not affected by the substance library.

Online Supplemental Figure S1. Control experiments in digitonin-permeabilized HeLa cells with a carrier-dependent control protein or p97. (A) shows that a carrier-dependent control protein requires cytosol for nuclear import into digitonin-permeabilized HeLa cells. Cells were incubated with digitonin (40 µg/ml) in transport buffer (TB) for 6 min on ice. After washing with TB the cells were incubated for 60 min at RT with GST-NLS-GFP control protein (0.8 µM) dissolved in 20 µl IM or complete import mix (CIM: IM supplemented with reticulocyte lysate (75% of volume) and an energy regeneration system (0.5 mM ATP, 0.5 mM GTP, 10 mM creatine phosphate, 30 U/ml creatine phosphokinase). Where indicated, the reaction (with CIM) was performed at 4°C (4°C). Additionally, CIM was supplemented with 0.5 mg/ml WGA (+WGA); or with 16 µM p97 (aa 45-462 of p97) (+p97tc), or with 0.8 U/ml apyrase (+apyrase). In (B) the cytosol-independent nuclear import of p97 is demonstrated. Import reaction without or with 0.8 µM His-tagged importin-β (p97) dissolved in IM. Where indicated, IM was supplemented with WGA at a concentration of 0.1 mg/ml (+WGA/100), or 0.25 mg/ml (+WGA/250). An anti-His antibody was used to detect p97. Bar, 30 µm.

Online Supplemental Figure S2. Immunoblotting results with the FG repeat-specific antibody 414 and bacterial lysates containing recombinant nucleoporins. Identification of bacterially expressed nucleoporin fusion proteins. Bacterial lysates containing maltose binding protein (MBP, lane 1) or FG repeat regions of Nup214 (residues 1549-2090, His-tagged), Nup62 (residues 1-308 fused to MBP), Nup153 (residues 333-618 fused to MBP) (lanes 2-4), or purified His-tagged tandem SH3 domains from FYB (lane 5) were resolved by 10% SDS-PAGE, transferred to a nitrocellulose membrane, and probed with FG repeat-specific monoclonal antibody 414 (Covance). Protein loading was 0.2% of the input used in Figure 7.

Table I. Nucleocytoplasmic translocation of unphosphorylated Stat proteins as determined by microinjection. a) Microinjection of recombinant Stat proteins. Listed is the amount of time required to reach equilibrium (min or h). b) Microinjection of Stat1 antibodies. Listed are the times required to observe maximal precipitation of endogenous Stat1 in the injected compartment. Each entry represents an averaged value observed by immunocytochemistry in 15-20 microinjected cells after fixation. GM, growth medium with 10% serum; EDM, serum- and glucose-free energy depletion medium with azide and deoxyglucose; n.a., not applicable; n.d., not done; AF, Alexa Fluor 594; OG, Oregon Green 488.

## Claims

1. Stat molecule occurring in the nucleus of a cutaneous T cell lymphoma cell line **characterized in that** at least one conserved hydrophobic amino acid on the surface of the N-domain of the Stat wild type is mutated.

2. Stat molecule according to claim 1, **characterized in that** the wild type of Stat2, Stat3, Stat5 or Stat6 is mutated.

3. Stat molecule according to claim 1 or 2, **characterized in that** the amino acids of the N-domain in position 81 and/or position 82 of Stat5 or in equivalent positions of homologous Stat proteins are mutated.

4. Stat molecule according to claim 3, **characterized in that** the amino acid in position 81 is substituted by alanine and/or the amino acid in position 82 is substituted by serine.

5. Stat molecules according to one of claims 1 to 4 which are dimerized.

6. A fusion protein which includes the Stat molecule according to one of claims 1 to 4 in combination with at least one marker structure.

7. A nucleic acid sequence which encodes the Stat molecule according to one of claims 1 to 4, or the fusion protein according to claim 6.

8. A vector, including the nucleic acid sequence according to claim 7.

9. A host cell, including the vector according to claim 8.

10. Use of the Stat molecule according to one of claims 1 to 5, the fusion protein according to claim 6, the nucleic acid sequence according to claim 7, the vector according to claim 8 or the host cell according to claim 9
- for expression of a target protein; or
- for investigating effects caused by different gene induction levels; or
- for screening of active substances which modify the transcription activity of target genes; or
- for the identification of binding partners; or
- for the production of a drug for the diagnosis and/or treatment of diseases associated with abnormal Stat induction of target genes.

11. Use of a Stat molecule derived from wild type Stat1, which is mutated in at least one conserved hydrophobic amino acid on the surface of the N-domain,
- for expression of a target protein; or
- for investigating effects caused by different gene induction levels; or
- for screening of active substances which modify the transcription activity of target genes; or
- for the identification of binding partners; or
- for the production of a drug for the diagnosis and/or treatment of diseases associated with abnormal Stat induction of target genes.

12. Method for overexpression of a target protein comprising the following steps:
- introduction of a vector according to claim 8 into a host cell or a cell-free system;
- introduction of a second vector harboring at least a single optimal GAS sequence followed by a target gene into a host cell or a cell-free system;
- induction of gene expressions; and
- maintenance of gene expressions over an appropriate period.

13. Method for investigating effects caused by different gene induction levels comprising the following steps:
- introduction of a vector according to claim 8 into a host cell;
- introduction of a second vector harboring a promoter sequence followed by a target gene into a host cell;
- induction of gene expressions;
- maintenance of gene expressions over an appropriate period; and
- monitoring effects on the host cell according to claim 9 by an appropriate detection procedure.

14. Method for screening of active substances which modify the transcription activity of target genes comprising the following steps:
- introduction of a vector according to claim 8 into a host cell or a cell-free system;
- introduction of a second vector harboring a promoter sequence followed by a target gene into a host cell or a cell-free system;
- induction of gene expressions;
- addition of a substance library;
- maintenance of the gene expressions over an appropriate period; and
- monitoring effects on the host cell according to claim 9 or the cell-free system by an appropriate detection procedure.

15. Method for the production of a drug for the diagnosis and/or treatment of diseases associated with abnormal Stat induction of target genes comprising the following steps:
- withdrawal of a tissue sample;
- cell disruption;
- separation of the intracellular protein extract;
- probing the protein extract with a high affinity molecule selectively recognizing the Stat mutant;
- gene therapy.

16. A kit comprising a Stat molecule according to one of claims 1 to 6, and/or synthetic analogues, modifications and pharmacologically active fragments thereof and an information about the using of the kit.

17. A kit comprising a nucleic acid sequence according to claim 7, and/or synthetic analogues, modifications and pharmacologically active fragments thereof and an information about the using of the kit.


### Annex to the application documents - subsequently filed sequences listing

SEQUENZPROTOKOLL

<110> Forschungsverbund Berlin e.V. (FMP)

<120> Hyperactive Stat molecules and their use in assays
      employing gene activation

<130> P286604EP1-La

<140> 04077629.6
<141> 2004-09-17

<160> 4

<170> PatentIn Ver. 2.1

<210> 1
<211> 750
<212> PRT
<213> Homo sapiens

<220>
<221> MUTAGEN
<222> (77)
<223> phenyalanine to alanine

<400> 1
Met Ser Gln Trp Tyr Glu Leu Gln Gln Leu Asp Ser Lys Phe Leu Glu
  1               5                  10                  15

Gln Val His Gln Leu Tyr Asp Asp Ser Phe Pro Met Glu Ile Arg Gln
             20                  25                  30

Tyr Leu Ala Gln Trp Leu Glu Lys Gln Asp Trp Glu His Ala Ala Asn
         35                  40                  45

Asp Val Ser Phe Ala Thr Ile Arg Phe His Asp Leu Leu Ser Gln Leu
     50                  55                  60

Asp Asp Gln Tyr Ser Arg Phe Ser Leu Glu Asn Asn Ala Leu Leu Gln
 65                  70                  75                  80

His Asn Ile Arg Lys Ser Lys Arg Asn Leu Gln Asp Asn Phe Gln Glu
                 85                  90                  95

Asp Pro Ile Gln Met Ser Met Ile Ile Tyr Ser Cys Leu Lys Glu Glu
            100                 105                 110

Arg Lys Ile Leu Glu Asn Ala Gln Arg Phe Asn Gln Ala Gln Ser Gly
        115                 120                 125

Asn Ile Gln Ser Thr Val Met Leu Asp Lys Gln Lys Glu Leu Asp Ser
    130                 135                 140

Lys Val Arg Asn Val Lys Asp Lys Val Met Cys Ile Glu His Glu Ile
145                 150                 155                 160

Lys Ser Leu Glu Asp Leu Gln Asp Glu Tyr Asp Phe Lys Cys Lys Thr
                165                 170                 175

Leu Gln Asn Arg Glu His Glu Thr Asn Gly Val Ala Lys Ser Asp Gln
            180                 185                 190

Lys Gln Glu Gln Leu Leu Leu Lys Lys Met Tyr Leu Met Leu Asp Asn
        195                 200                 205

Lys Arg Lys Glu Val Val His Lys Ile Ile Glu Leu Leu Asn Val Thr
    210                 215                 220

Glu Leu Thr Gln Asn Ala Leu Ile Asn Asp Glu Leu Val Glu Trp Lys
225                 230                 235                 240

Arg Arg Gln Gln Ser Ala Cys Ile Gly Gly Pro Pro Asn Ala Cys Leu
                245                 250                 255

Asp Gln Leu Gln Asn Trp Phe Thr Ile Val Ala Glu Ser Leu Gln Gln
            260                 265                 270

Val Arg Gln Gln Leu Lys Lys Leu Glu Glu Leu Glu Gln Lys Tyr Thr
        275                 280                 285

Tyr Glu His Asp Pro Ile Thr Lys Asn Lys Gln Val Leu Trp Asp Arg
    290                 295                 300

Thr Phe Ser Leu Phe Gln Gln Leu Ile Gln Ser Ser Phe Val Val Glu
305                 310                 315                 320

Arg Gln Pro Cys Met Pro Thr His Pro Gln Arg Pro Leu Val Leu Lys
                325                 330                 335

Thr Gly Val Gln Phe Thr Val Lys Leu Arg Leu Leu Val Lys Leu Gln
            340                 345                 350

Glu Leu Asn Tyr Asn Leu Lys Val Lys Val Leu Phe Asp Lys Asp Val
        355                 360                 365

Asn Glu Arg Asn Thr Val Lys Gly Phe Arg Lys Phe Asn Ile Leu Gly
    370                 375                 380

Thr His Thr Lys Val Met Asn Met Glu Glu Ser Thr Asn Gly Ser Leu
385                 390                 395                 400

Ala Ala Glu Phe Arg His Leu Gln Leu Lys Glu Gln Lys Asn Ala Gly
                405                 410                 415

Thr Arg Thr Asn Glu Gly Pro Leu Ile Val Thr Glu Glu Leu His Ser
            420                 425                 430

Leu Ser Phe Glu Thr Gln Leu Cys Gln Pro Gly Leu Val Ile Asp Leu
        435                 440                 445

Glu Thr Thr Ser Leu Pro Val Val Val Ile Ser Asn Val Ser Gln Leu
    450                 455                 460

Pro Ser Gly Trp Ala Ser Ile Leu Trp Tyr Asn Met Leu Val Ala Glu
465                 470                 475                 480

Pro Arg Asn Leu Ser Phe Phe Leu Thr Pro Pro Cys Ala Arg Trp Ala
                485                 490                 495

Gln Leu Ser Glu Val Leu Ser Trp Gln Phe Ser Ser Val Thr Lys Arg
            500                 505                 510

Gly Leu Asn Val Asp Gln Leu Asn Met Leu Gly Glu Lys Leu Leu Gly
        515                 520                 525

Pro Asn Ala Ser Pro Asp Gly Leu Ile Pro Trp Thr Arg Phe Cys Lys
    530                 535                 540

Glu Asn Ile Asn Asp Lys Asn Phe Pro Phe Trp Leu Trp Ile Glu Ser
545                 550                 555                 560

Ile Leu Glu Leu Ile Lys Lys His Leu Leu Pro Leu Trp Asn Asp Gly
                565                 570                 575

Cys Ile Met Gly Phe Ile Ser Lys Glu Arg Glu Arg Ala Leu Leu Lys
            580                 585                 590

Asp Gln Gln Pro Gly Thr Phe Leu Leu Arg Phe Ser Glu Ser Ser Arg
        595                 600                 605

Glu Gly Ala Ile Thr Phe Thr Trp Val Glu Arg Ser Gln Asn Gly Gly
    610                 615                 620

Glu Pro Asp Phe His Ala Val Glu Pro Tyr Thr Lys Lys Glu Leu Ser
625                 630                 635                 640

Ala Val Thr Phe Pro Asp Ile Ile Arg Asn Tyr Lys Val Met Ala Ala
                645                 650                 655

Glu Asn Ile Pro Glu Asn Pro Leu Lys Tyr Leu Tyr Pro Asn Ile Asp
            660                 665                 670

Lys Asp His Ala Phe Gly Lys Tyr Tyr Ser Arg Pro Lys Glu Ala Pro
        675                 680                 685

Glu Pro Met Glu Leu Asp Gly Pro Lys Gly Thr Gly Tyr Ile Lys Thr
    690                 695                 700

Glu Leu Ile Ser Val Ser Glu Val His Pro Ser Arg Leu Gln Thr Thr
705                 710                 715                 720

Asp Asn Leu Leu Pro Met Ser Pro Glu Glu Phe Asp Glu Val Ser Arg
                725                 730                 735

Ile Val Gly Ser Val Glu Phe Asp Ser Met Met Asn Thr Val
            740                 745                 750

<210> 2
<211> 712
<212> PRT
<213> Homo sapiens

<220>
<221> MUTAGEN
<222> (77)
<223> phenylalanine to alanine

<400> 2
Met Ser Gln Trp Tyr Glu Leu Gln Gln Leu Asp Ser Lys Phe Leu Glu
1               5                   10                  15

Gln Val His Gln Leu Tyr Asp Asp Ser Phe Pro Met Glu Ile Arg Gln
            20                  25                  30

Tyr Leu Ala Gln Trp Leu Glu Lys Gln Asp Trp Glu His Ala Ala Asn
        35                  40                  45

Asp Val Ser Phe Ala Thr Ile Arg Phe His Asp Leu Leu Ser Gln Leu
    50                  55                  60

Asp Asp Gln Tyr Ser Arg Phe Ser Leu Glu Asn Asn Ala Leu Leu Gln
65                  70                  75                  80

His Asn Ile Arg Lys Ser Lys Arg Asn Leu Gln Asp Asn Phe Gln Glu
                85                  90                  95

Asp Pro Ile Gln Met Ser Met Ile Ile Tyr Ser Cys Leu Lys Glu Glu
            100                 105                 110

Arg Lys Ile Leu Glu Asn Ala Gln Arg Phe Asn Gln Ala Gln Ser Gly
        115                 120                 125

Asn Ile Gln Ser Thr Val Met Leu Asp Lys Gln Lys Glu Leu Asp Ser
    130                 135                 140

Lys Val Arg Asn Val Lys Asp Lys Val Met Cys Ile Glu His Glu Ile
145                 150                 155                 160

Lys Ser Leu Glu Asp Leu Gln Asp Glu Tyr Asp Phe Lys Cys Lys Thr
                165                 170                 175

Leu Gln Asn Arg Glu His Glu Thr Asn Gly Val Ala Lys Ser Asp Gln
            180                 185                 190

Lys Gln Glu Gln Leu Leu Leu Lys Lys Met Tyr Leu Met Leu Asp Asn
        195                 200                 205

Lys Arg Lys Glu Val Val His Lys Ile Ile Glu Leu Leu Asn Val Thr
    210                 215                 220

Glu Leu Thr Gln Asn Ala Leu Ile Asn Asp Glu Leu Val Glu Trp Lys
225                 230                 235                 240

Arg Arg Gln Gln Ser Ala Cys Ile Gly Gly Pro Pro Asn Ala Cys Leu
                245                 250                 255

Asp Gln Leu Gln Asn Trp Phe Thr Ile Val Ala Glu Ser Leu Gln Gln
            260                 265                 270

Val Arg Gln Gln Leu Lys Lys Leu Glu Glu Leu Glu Gln Lys Tyr Thr
        275                 280                 285

Tyr Glu His Asp Pro Ile Thr Lys Asn Lys Gln Val Leu Trp Asp Arg
    290                 295                 300

Thr Phe Ser Leu Phe Gln Gln Leu Ile Gln Ser Ser Phe Val Val Glu
305                 310                 315                 320

Arg Gln Pro Cys Met Pro Thr His Pro Gln Arg Pro Leu Val Leu Lys
                325                 330                 335

Thr Gly Val Gln Phe Thr Val Lys Leu Arg Leu Leu Val Lys Leu Gln
            340                 345                 350

Glu Leu Asn Tyr Asn Leu Lys Val Lys Val Leu Phe Asp Lys Asp Val
        355                 360                 365

Asn Glu Arg Asn Thr Val Lys Gly Phe Arg Lys Phe Asn Ile Leu Gly
    370                 375                 380

Thr His Thr Lys Val Met Asn Met Glu Glu Ser Thr Asn Gly Ser Leu
385                 390                 395                 400

Ala Ala Glu Phe Arg His Leu Gln Leu Lys Glu Gln Lys Asn Ala Gly
                405                 410                 415

Thr Arg Thr Asn Glu Gly Pro Leu Ile Val Thr Glu Glu Leu His Ser
            420                 425                 430

Leu Ser Phe Glu Thr Gln Leu Cys Gln Pro Gly Leu Val Ile Asp Leu
        435                 440                 445

Glu Thr Thr Ser Leu Pro Val Val Val Ile Ser Asn Val Ser Gln Leu
    450                 455                 460

Pro Ser Gly Trp Ala Ser Ile Leu Trp Tyr Asn Met Leu Val Ala Glu
465                 470                 475                 480

Pro Arg Asn Leu Ser Phe Phe Leu Thr Pro Pro Cys Ala Arg Trp Ala
                485                 490                 495

Gln Leu Ser Glu Val Leu Ser Trp Gln Phe Ser Ser Val Thr Lys Arg
            500                 505                 510

Gly Leu Asn Val Asp Gln Leu Asn Met Leu Gly Glu Lys Leu Leu Gly
        515                 520                 525

Pro Asn Ala Ser Pro Asp Gly Leu Ile Pro Trp Thr Arg Phe Cys Lys
    530                 535                 540

Glu Asn Ile Asn Asp Lys Asn Phe Pro Phe Trp Leu Trp Ile Glu Ser
545                 550                 555                 560

Ile Leu Glu Leu Ile Lys Lys His Leu Leu Pro Leu Trp Asn Asp Gly
                565                 570                 575

Cys Ile Met Gly Phe Ile Ser Lys Glu Arg Glu Arg Ala Leu Leu Lys
            580                 585                 590

Asp Gln Gln Pro Gly Thr Phe Leu Leu Arg Phe Ser Glu Ser Ser Arg
        595                 600                 605

Glu Gly Ala Ile Thr Phe Thr Trp Val Glu Arg Ser Gln Asn Gly Gly
    610                 615                 620

Glu Pro Asp Phe His Ala Val Glu Pro Tyr Thr Lys Lys Glu Leu Ser
625                 630                 635                 640

Ala Val Thr Phe Pro Asp Ile Ile Arg Asn Tyr Lys Val Met Ala Ala
                645                 650                 655

Glu Asn Ile Pro Glu Asn Pro Leu Lys Tyr Leu Tyr Pro Asn Ile Asp
            660                 665                 670

Lys Asp His Ala Phe Gly Lys Tyr Tyr Ser Arg Pro Lys Glu Ala Pro
        675                 680                 685

Glu Pro Met Glu Leu Asp Gly Pro Lys Gly Thr Gly Tyr Ile Lys Thr
    690                 695                 700

Glu Leu Ile Ser Val Ser Glu Val
705                 710

<210> 3
<211> 700
<212> PRT
<213> Homo sapiens

<220>
<221> MUTAGEN
<222> (81)
<223> phenylalanine to alanine

<220>
<221> NON_TER
<222> (700)

<400> 3
Met Ala Gly Trp Ile Gln Ala Gln Gln Leu Gln Gly Asp Ala Leu Arg
1               5                   10                  15

Gln Met Gln Val Leu Tyr Gly Gln His Phe Pro Ile Glu Val Arg His
            20                  25                  30

Tyr Leu Ala Gln Trp Ile Glu Ser Gln Pro Trp Asp Ala Ile Asp Leu
        35                  40                  45

Asp Asn Pro Gln Asp Arg Ala Gln Ala Thr Gln Leu Leu Glu Gly Leu
    50                  55                  60

Val Gln Glu Leu Gln Lys Lys Ala Glu His Gln Val Gly Glu Asp Gly
65                  70                  75                  80

Ala Leu Leu Lys Ile Lys Leu Gly His Tyr Ala Thr Gln Leu Gln Lys
                85                  90                  95

Thr Tyr Asp Arg Cys Pro Leu Glu Leu Val Arg Cys Ile Arg His Ile
            100                 105                 110

Leu Tyr Asn Glu Gln Arg Leu Val Arg Glu Ala Asn Asn Cys Ser Ser
        115                 120                 125

Pro Ala Gly Ile Leu Val Asp Ala Met Ser Gln Lys His Leu Gln Ile
    130                 135                 140

Asn Gln Thr Phe Glu Glu Leu Arg Leu Val Thr Gln Asp Thr Glu Asn
145                 150                 155                 160

Glu Leu Lys Lys Leu Gln Gln Thr Gln Glu Tyr Phe Ile Ile Gln Tyr
                165                 170                 175

Gln Glu Ser Leu Arg Ile Gln Ala Gln Phe Ala Gln Leu Ala Gln Leu
            180                 185                 190

Ser Pro Gln Glu Arg Leu Ser Arg Glu Thr Ala Leu Gln Gln Lys Gln
        195                 200                 205

Val Ser Leu Glu Ala Trp Leu Gln Arg Glu Ala Gln Thr Leu Gln Gln
    210                 215                 220

Tyr Arg Val Glu Leu Ala Glu Lys His Gln Lys Thr Leu Gln Leu Leu
225                 230                 235                 240

Arg Lys Gln Gln Thr Ile Ile Leu Asp Asp Glu Leu Ile Gln Trp Lys
                245                 250                 255

Arg Arg Gln Gln Leu Ala Gly Asn Gly Gly Pro Pro Glu Gly Ser Leu
            260                 265                 270

Asp Val Leu Gln Ser Trp Cys Glu Lys Leu Ala Glu Ile Ile Trp Gln
        275                 280                 285

Asn Arg Gln Gln Ile Arg Arg Ala Glu His Leu Cys Gln Gln Leu Pro
    290                 295                 300

Ile Pro Gly Pro Val Glu Glu Met Leu Ala Glu Val Asn Ala Thr Ile
305                 310                 315                 320

Thr Asp Ile Ile Ser Ala Leu Val Thr Ser Thr Phe Ile Ile Glu Lys
                325                 330                 335

Gln Pro Pro Gln Val Leu Lys Thr Gln Thr Lys Phe Ala Ala Thr Val
            340                 345                 350

Arg Leu Leu Val Gly Gly Lys Leu Asn Val His Met Asn Pro Pro Gln
        355                 360                 365

Val Lys Ala Thr Ile Ile Ser Glu Gln Gln Ala Lys Ser Leu Leu Lys
    370                 375                 380

Asn Glu Asn Thr Arg Asn Glu Cys Ser Gly Glu Ile Leu Asn Asn Cys
385                 390                 395                 400

Cys Val Met Glu Tyr His Gln Ala Thr Gly Thr Leu Ser Ala His Phe
                405                 410                 415

Arg Asn Met Ser Leu Lys Arg Ile Lys Arg Ala Asp Arg Arg Gly Ala
            420                 425                 430

Glu Ser Val Thr Glu Glu Lys Phe Thr Val Leu Phe Glu Ser Gln Phe
        435                 440                 445

Ser Val Gly Ser Asn Glu Leu Val Phe Gln Val Lys Thr Leu Ser Leu
    450                 455                 460

Pro Val Val Val Ile Val His Gly Ser Gln Asp His Asn Ala Thr Ala
465                 470                 475                 480

Thr Val Leu Trp Asp Asn Ala Phe Ala Glu Pro Gly Arg Val Pro Phe
                485                 490                 495

Ala Val Pro Asp Lys Val Leu Trp Pro Gln Leu Cys Glu Ala Leu Asn
            500                 505                 510

Met Lys Phe Lys Ala Glu Val Gln Ser Asn Arg Gly Leu Thr Lys Glu
        515                 520                 525

Asn Leu Val Phe Leu Ala Gln Lys Leu Phe Asn Asn Ser Ser Ser His
    530                 535                 540

Leu Glu Asp Tyr Ser Gly Leu Ser Val Ser Trp Ser Gln Phe Asn Arg
545                 550                 555                 560

Glu Asn Leu Pro Gly Trp Asn Tyr Thr Phe Trp Gln Trp Phe Asp Gly
                565                 570                 575

Val Met Glu Val Leu Lys Lys His His Lys Pro His Trp Asn Asp Gly
            580                 585                 590

Ala Ile Leu Gly Phe Val Asn Lys Gln Gln Ala His Asp Leu Leu Ile
        595                 600                 605

Asn Lys Pro Asp Gly Thr Phe Leu Leu Arg Phe Ser Asp Ser Glu Ile
    610                 615                 620

Gly Gly Ile Thr Ile Ala Trp Lys Phe Asp Ser Pro Glu Arg Asn Leu
625                 630                 635                 640

Trp Asn Leu Lys Pro Phe Thr Thr Arg Asp Phe Ser Ile Arg Ser Leu
                645                 650                 655

Ala Asp Arg Leu Gly Asp Leu Ser Tyr Leu Ile Tyr Val Phe Pro Asp
            660                 665                 670

Arg Pro Lys Asp Glu Val Phe Ser Lys Tyr Tyr Thr Pro Val Leu Ala
        675                 680                 685

Lys Ala Val Asp Gly Tyr Val Lys Pro Gln Ile Lys
    690                 695                 700

<210> 4
<211> 715
<212> PRT
<213> Homo sapiens

<220>
<221> MUTAGEN
<222> (81)
<223> phenylalanine to alanine

<220>
<221> NON_TER
<222> (715)

<400> 4
Met Ala Val Trp Ile Gln Ala Gln Gln Leu Gln Gly Glu Ala Leu His

1                   5                   10                  15

Gln Met Gln Ala Leu Tyr Gly Gln His Phe Pro Ile Glu Val Arg His
                20                  25                  30

Tyr Leu Ser Gln Trp Ile Glu Ser Gln Ala Trp Asp Ser Val Asp Leu
            35                  40                  45

Asp Asn Pro Gln Glu Asn Ile Lys Ala Thr Gln Leu Leu Glu Gly Leu
    50                  55                  60

Val Gln Glu Leu Gln Lys Lys Ala Glu His Gln Val Gly Glu Asp Gly
65                  70                  75                  80

Ala Leu Leu Lys Ile Lys Leu Gly His Tyr Ala Thr Gln Leu Gln Asn
                85                  90                  95

Thr Tyr Asp Arg Cys Pro Met Glu Leu Val Arg Cys Ile Arg His Ile
            100                 105                 110

Leu Tyr Asn Glu Gln Arg Leu Val Arg Glu Ala Asn Asn Gly Ser Ser
        115                 120                 125

Pro Ala Gly Ser Leu Ala Asp Ala Met Ser Gln Lys His Leu Gln Ile
    130                 135                 140

Asn Gln Thr Phe Glu Glu Leu Arg Leu Val Thr Gln Asp Thr Glu Asn
145                 150                 155                 160

Glu Leu Lys Lys Leu Gln Gln Thr Gln Glu Tyr Phe Ile Ile Gln Tyr
                165                 170                 175

Gln Glu Ser Leu Arg Ile Gln Ala Gln Phe Gly Pro Leu Ala Gln Leu
            180                 185                 190

Ser Pro Gln Glu Arg Leu Ser Arg Glu Thr Ala Leu Gln Gln Lys Gln
        195                 200                 205

Val Ser Leu Glu Ala Trp Leu Gln Arg Glu Ala Gln Thr Leu Gln Gln
    210                 215                 220

Tyr Arg Val Glu Leu Ala Glu Lys His Gln Lys Thr Leu Gln Leu Leu
225                 230                 235                 240

Arg Lys Gln Gln Thr Ile Ile Leu Asp Asp Glu Leu Ile Gln Trp Lys
                245                 250                 255

Arg Arg Gln Gln Leu Ala Gly Asn Gly Gly Pro Pro Glu Gly Ser Leu

            260                 265                 270

Asp Val Leu Gln Ser Trp Cys Glu Lys Leu Ala Glu Ile Ile Trp Gln
        275                 280                 285

Asn Arg Gln Gln Ile Arg Arg Ala Glu His Leu Cys Gln Gln Leu Pro
    290                 295                 300

Ile Pro Gly Pro Val Glu Glu Met Leu Ala Glu Val Asn Ala Thr Ile
305                 310                 315                 320

Thr Asp Ile Ile Ser Ala Leu Val Thr Ser Thr Phe Ile Ile Glu Lys
                325                 330                 335

Gln Pro Pro Gln Val Leu Lys Thr Gln Thr Lys Phe Ala Ala Thr Val
            340                 345                 350

Arg Leu Leu Val Gly Gly Lys Leu Asn Val His Met Asn Pro Pro Gln
        355                 360                 365

Val Lys Ala Thr Ile Ile Ser Glu Gln Gln Ala Lys Ser Leu Leu Lys
    370                 375                 380

Asn Glu Asn Thr Arg Asn Asp Tyr Ser Gly Glu Ile Leu Asn Asn Cys
385                 390                 395                 400

Cys Val Met Glu Tyr His Gln Ala Thr Gly Thr Leu Ser Ala His Phe
                405                 410                 415

Arg Asn Met Ser Leu Lys Arg Ile Lys Arg Ser Asp Arg Arg Gly Ala
            420                 425                 430

Glu Ser Val Thr Glu Glu Lys Phe Thr Ile Leu Phe Glu Ser Gln Phe
        435                 440                 445

Ser Val Gly Gly Asn Glu Leu Val Phe Gln Val Lys Thr Leu Ser Leu
    450                 455                 460

Pro Val Val Val Ile Val His Gly Ser Gln Asp Asn Asn Ala Thr Ala
465                 470                 475                 480

Thr Val Leu Trp Asp Asn Ala Phe Ala Glu Pro Gly Arg Val Pro Phe
                485                 490                 495

Ala Val Pro Asp Lys Val Leu Trp Pro Gln Leu Cys Glu Ala Leu Asn
            500                 505                 510

Met Lys Phe Lys Ala Glu Val Gln Ser Asn Arg Gly Leu Thr Lys Glu

515                 520                 525

Asn Leu Val Phe Leu Ala Gln Lys Leu Phe Asn Asn Ser Ser Ser His
    530                 535                 540

Leu Glu Asp Tyr Ser Gly Leu Ser Val Ser Trp Ser Gln Phe Asn Arg
545                 550                 555                 560

Glu Asn Leu Pro Gly Arg Asn Tyr Thr Phe Trp Gln Trp Phe Asp Gly
                565                 570                 575

Val Met Glu Val Leu Lys Lys His Leu Lys Pro His Trp Asn Asp Gly
            580                 585                 590

Ala Ile Leu Gly Phe Val Asn Lys Gln Gln Ala His Asp Leu Leu Ile
        595                 600                 605

Asn Lys Pro Asp Gly Thr Phe Leu Leu Arg Phe Ser Asp Ser Glu Ile
    610                 615                 620

Gly Gly Ile Thr Ile Ala Trp Lys Phe Asp Ser Gln Glu Arg Met Phe
625                 630                 635                 640

Trp Asn Leu Met Pro Phe Thr Thr Arg Asp Phe Ser Ile Arg Ser Leu
                645                 650                 655

Ala Asp Arg Leu Gly Asp Leu Asn Tyr Leu Ile Tyr Val Phe Pro Asp
            660                 665                 670

Arg Pro Lys Asp Glu Val Tyr Ser Lys Tyr Tyr Thr Pro Val Pro Cys
        675                 680                 685

Glu Ser Ala Thr Ala Lys Ala Val Asp Gly Tyr Val Lys Pro Gln Ile
    690                 695                 700

Lys Gln Val Val Pro Glu Phe Val Asn Ala Ser
705                 710                 715